(19) Europäisches Patentamt
European Patent Office
Office européen des brevets

(11) **EP 1 513 466 B1**

(12) **EUROPÄISCHE PATENTSCHRIFT**

(45) Veröffentlichungstag und Bekanntmachung des Hinweises auf die Patenterteilung:
**21.01.2009 Patentblatt 2009/04**

(21) Anmeldenummer: **02787734.9**

(22) Anmeldetag: **19.11.2002**

(51) Int Cl.:
***A61F 2/00*** *(2006.01)*

(86) Internationale Anmeldenummer:
**PCT/EP2002/012963**

(87) Internationale Veröffentlichungsnummer:
**WO 2003/043534 (30.05.2003 Gazette 2003/22)**

(54) **VERSCHLUSSSYSTEM MIT ELEKTRONISCHER STEUERUNG**

CLOSING SYSTEM WITH ELECTRONIC CONTROL

SYSTEME DE FERMETURE AVEC COMMANDE ELECTRONIQUE

(84) Benannte Vertragsstaaten:
**AT BE BG CH CY CZ DE DK EE ES FI FR GB GR IE IT LI LU MC NL PT SE SK TR**

(30) Priorität: **20.11.2001 DE 10156558
27.08.2002 DE 10239309**

(43) Veröffentlichungstag der Anmeldung:
**16.03.2005 Patentblatt 2005/11**

(73) Patentinhaber:
• **Jocham, Dieter
23568 Lübeck (DE)**
• **Wassermann, Helmut
81739 München (DE)**

(72) Erfinder:
• **WASSERMANN, Helmut
81739 München (DE)**

• **SCHOSTEK, Sebastian
72074 Tübingen (DE)**
• **HO, Chi-Nghia
80995 München (DE)**

(74) Vertreter: **Schneider, Michael et al
HAMMONDS
Karl-Scharnagl-Ring 7
80539 München (DE)**

(56) Entgegenhaltungen:
**EP-A- 0 409 592          WO-A-00/15140
WO-A-01/45487          WO-A-01/50833
US-A- 4 417 567          US-A- 4 571 749
US-A- 4 721 509          US-A- 6 074 341
US-A- 6 135 945**

**Beschreibung**

Die folgende Erfindung betrifft ein Verschlusssystem mit einem geeignetes Verfahren zum wahlweisen Öffnen und Schließen eines rohrförmigen Körperorgans.

Aus der DE 43 31 658 ist eine implantierbare Vorrichtung zum wahlweisen Öffnen und Schließen von rohrförmigen Körperorganen bekannt, wobei ein in das rohrförmige Körperorgan einführbarer länglicher Ventilkörper vorgesehen ist. Der Ventilkörper weist eine Absperreinrichtung auf, die wahlweise verschlossen und freigegeben werden kann. Dazu besitzt der Ventilkörper einen elastischen Schlauchabschnitt, in welchem ein Blähkörper angeordnet ist, der durch ein Fluid aufblähbar ist und dann das Lumen des Schlauchabschnitts verschließt. Sowohl das Öffnen als auch das Schließen erfolgt durch eine manuelle Handhabung, d.h. das Aufblähen des Blähkörpers erfolgt durch eine manuelle Betätigung einer Pumpe und das Öffnen erfolgt durch ein manuelles Betätigen eines Schalters. Grundsätzlich ist jedoch bei dem bekannten System darauf zu achten, dass der Blähkörper nicht derart stark aufgebläht wird, dass zum Einen sich das Körperorgan in Folge eines häufigen Öffnens und Schließens ebenfalls vergrößert und ausdehnt, und zum Anderen der durch den Blähkörper erzeugte Druck, die Durchblutung des Körperorgans unterbinden würde und somit das Gewebe des Körperorgans absterben würde. Diese Vorgaben erfordern somit, dass das Verschließen des Körperorgans mit dem bekannten System nur innerhalb eines beschränkten Druckbereiches möglich ist, wobei hingegen entstehende Druckspitzen bzw. kurzzeitig auftretende Druckbelastungen bzw. -erhöhungen in dem Körperorgan, einen sicheren Verschluss des Körperorgans nicht sicherstellen kann, da das bekannte System nicht geeignet ist, kurzzeitig nachgeführt zu werden.

WO-A-01/50833 offenbart eine Behandlungsvorrichtung für Harninkontinenz mit einer einstellbaren Drosselungsvorrichtung zum Verschließen des Harndurchgangs durch die Harnröhre. Es ist ein Drucksensor implantiert zum Empfangen des Druckes auf die Drosselungsvorrichtung, wobei eine Steuerungseinheit die Drosselungsvorrichtung steuert, um die Harnröhre als Reaktion darauf freizugeben, dass der Drucksensor einen abnormalen hohen Druck empfängt.

Eine Aufgabe der vorliegenden Erfindung ist es deshalb, das bekannte Verschlusssystem bzw. das bekannte Verfahren zum wahlweisen Öffnen und Schließen eines rohrförmigen Körperorgans derart weiterzubilden, dass in dem zu verschließenden Körperorgan auftretende Druckerhöhungen kurzzeitig entgegengewirkt wird und eine Nekrosegefahr nahezu ausgeschlossen ist.

Eine weitere Aufgabe der vorliegenden Erfindung ist es, ein Verschlusssystem bereitzustellen, das jederzeit Kontinenz sicherstellt.

Gelöst werden die Aufgaben mit den Merkmalen der Ansprüche 1 und 5.

Anmeldungsgemäß weist das Verschlusssystem zum wahlweisen Öffnen und Schließen eines rohrförmigen Körperorgans die Bereitstellung eines Schließelementes und ein das Schließelement steuerndes Regelungssystem auf, wobei das Regelungssystem einen ersten Zustand des Verschlusssystems einstellt und eine Abweichung von dem ersten Zustand selbstregulierend auf den ersten Zustand zurückführt. Mit dieser anmeldungsgemäßen Maßnahme wird erreicht, dass bei auftretenden Druckspitzen bzw. -belastungen durch Husten, Niesen, Lachen oder Bücken weiterhin die erforderlichen Aufgaben des Verschlusssystems erfüllen kann. Mit dieser anmeldungsgemäßen Maßnahme kann beispielsweise, wenn das Verschlusssystem bei der Urethra angeregt wird, ein Sphinkter-Ersatzsystem zur Verfügung gestellt werden, welches die Funktion des äußeren Schließmuskels der Harnröhre beim erwachsenen Menschen ersetzt. Das anmeldungsgemäße Verschlusssystem kann somit als implantierbares adaptives feinsensorisches Sphinkter-Ersatzsystem eingesetzt werden. Dadurch, dass lediglich kurzzeitig durch Druckspitzen ein erster Zustand des Regelungssystems verlassen wird, wird die Nekrosegefahr vermindert bzw. Nekroseerscheinungen können verhindert werden. Mit dem anmeldungsgemäßen Verschlusssystem wird somit vermieden, dass ein konstant hoher Schließdruck auf das Körperorgan bzw. die Urethra einwirkt, wodurch das umliegende Gewebe des Körperorgans zur Nekrose bzw. zu Entzündungen führen würde. Da das anmeldungsgemäße Verschlusssystem eine einfach konzipierte Selbstregelung bereitstellt, ist auch das Implantieren in den menschlichen Körper unbedenklich. Darüber hinaus ist es möglich, die Miktion manuell durch dieses leicht bedienbare Verschlusssystem zu steuern, wobei auch die Steuerung des Verschlusssystems automatisch durch einen Abgriff der neurologischen Signale direkt an die Nervenbahn des Körperorgans im Falle der Urethra der Sphinkter Urethrer mit Hilfe einer künstlichen Synapsis möglich ist. Mit den anmeldungsgemäßen Verschlusssystem ist es darüber hinaus möglich, dass aufgrund der Einfachheit des Verschlusssystems der Energieverbrauch gering gehalten ist und somit eine lange Lebensdauer gegeben ist. Mit dem anmeldungsgemäßen Verschlusssystem erhält der Patient durch die Sicherstellung seiner Kontinenz mit diesem Implantat ein großes Maß an Lebensqualität zurück und wird durch die Wartungsfreiheit des Systems nicht in seinem Aktionsradius eingeschränkt.

Weitere vorteilhafte Ausgestaltungen der vorliegenden Erfindung sind Gegenstand der Unteransprüche.

Ist das Regelungssystem mit dem Schließelement als geschlossener Kreislauf ausgestaltet, so ist eine separate Zuführung des Übertragungsmediums, beispielsweise Hydraulikmittel nicht erforderlich.

Mit den dargelegten Merkmalen wird das selbstregulierende Verschlusssystem auf einfache Weise umgesetzt, so dass das Verschlusssystem als solches einfach zu implantieren ist und nur einen geringen Energieverbrauch aufweist. Die Selbstregulierung wird insbesondere durch das wechselseitige Öffnen und Schließen der Absperrventile erreicht. An dieser Stelle soll jedoch darauf hingewiesen werden, dass auch an Stelle der Pumpeinrichtung und erstes Absperrventil auch eine schnellschaltende Pumpe bzw. schnellschaltender Aktor eingesetzt werden kann.

Um die Selbstregulierung je nach Körperorgan bestmöglich einstellen zu können, d.h. der Schließdruck auf einen bestimmten Schwellenwert gehalten wird und diesbezüglich ein entsprechender Arbeitsdruck eingestellt werden kann, so sind unterschiedlich Sensorelemente vorgesehen, die an geeigneter Positionierung optimal die selbstregulierende Maßnahme herbei= führt.

Weitere vorteilhafte Ausgestaltungen sind Gegenstand der übrigen Unteransprüche.

Anhand nachfolgender Zeichnung soll eine bevorzugte Ausführungsform des Anmeldungsgegenstandes beschrieben werden.

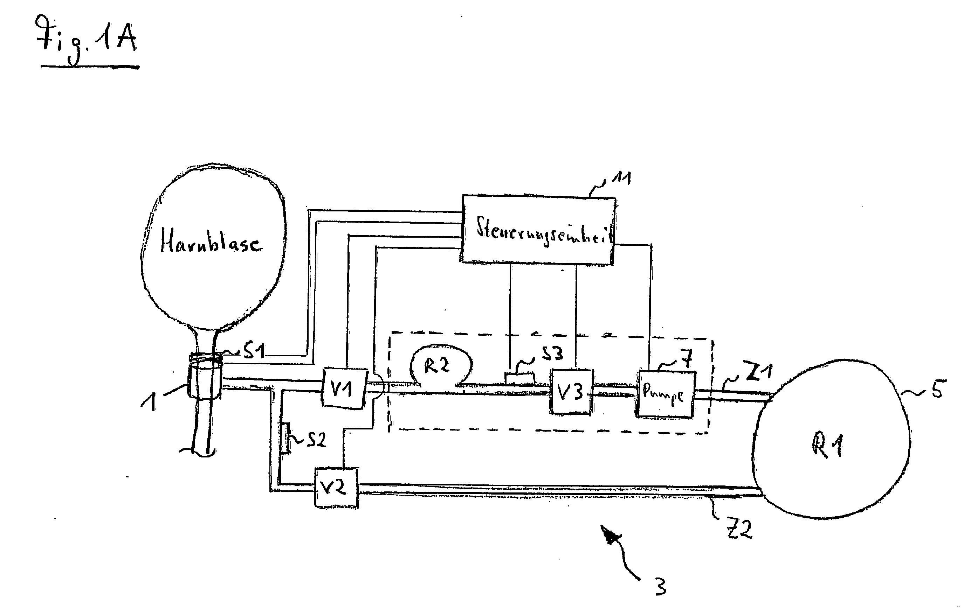

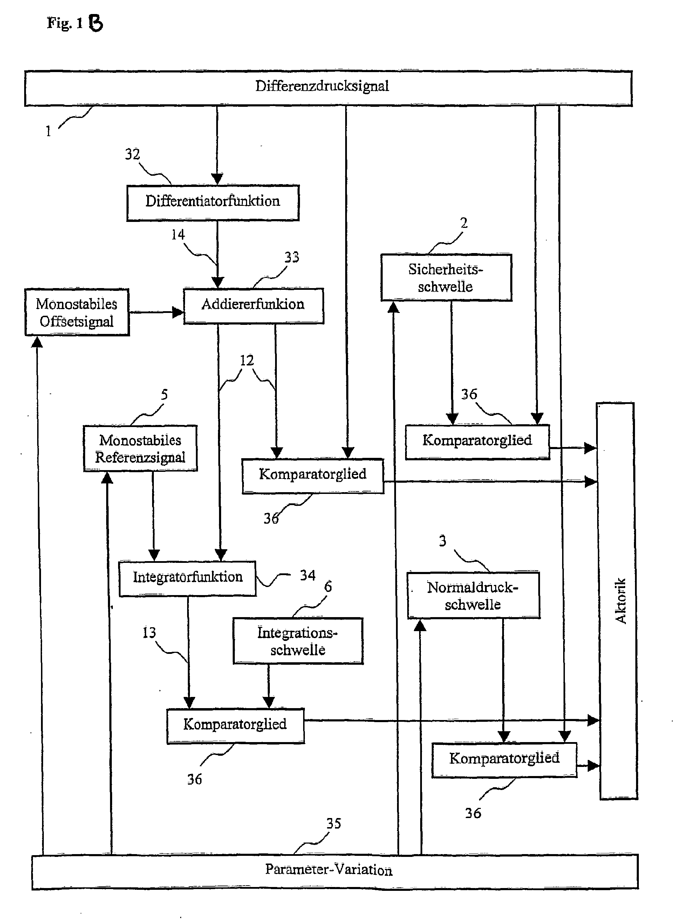

In Fig. 1A ist das Verschlusssystem gezeigt, welches in diesem Fall eine Urethra als Körperorgan wahlweise öffnet und schließt. Die Urethra weist bei dem anmeldungsgemäßen Verschlusssystem ein Schließelement 1 auf, welches in dieser Ausführungsform hydraulisch ansteuerbar ist. Bei dieser Gelegenheit wird darauf hingewiesen, dass jede andere Ansteuerung ebenfalls denkbar ist. An das Schließelement 1 ist ein Regelungssystem 3, in diesem Fall hydraulisch, anschließbar. Das Regelungssystem 3 dient dazu, einen ersten Zustand des Verschlusssystems, beispielsweise einen Schließzustand des Schließelements 1, einzustellen. Hierzu weist das Regelungssystem 3 bei der hydraulischen Ausführung, ein erstes Reservoir 5 auf, um über eine Pumpeinrichtung 7 einen bestimmten Druck, sogenannter Schließdruck am Schließelement 1 aufzubauen. Die an das Schließelement ankommende Verbindungsleitung teilt sich in eine erste Zuführungsleitung Z1 und eine zweite Zuführungsleitung Z2 auf, wobei die erste Zuführungsleitung die Pumpeinrichtung 7 und vorzugsweise ein erstes Absperrventil V1 aufweist und die zweite Zuführungsleitung ein zweites Absperrventil V2 aufweist, wobei sowohl die erste Zuführungsleitung als auch die zweite Zuführungsleitung mit dem ersten Reservoir 5 verbunden ist. Herkömmlicherweise kann das anmeldungsgemäße Verschlusssystem auch lediglich als eine Vorrichtung zum wahlweisen Öffnen und Schließen eines Körperorgans, in diesem Fall der Urethra herangezogen werden. Zum Betreiben des wahlweisen Öffnens und Schließens ist es von Vorteil, wenn die Pumpeinrichtung 7 , das erste Absperrventil V1 und das zweite Absperrventil V2 mit einer Steuerungseinheit 11 verbunden sind, welche das herkömmliche Öffnen und Schließen des Schließelements 1 übernimmt. Das anmeldungsgemäße Verschlusssystem weist ferner eine erste Sensoreinheit S1, welche am Schließelement 1, des rohrförmigen Körperorgans vorzugsweise stromaufwärts vorgesehen ist, eine zweite Sensoreinheit S2, welche den Druck in der Verbindungsleitung misst und eine dritte Sensoreinheit S3 an der Pumpeinrichtung 7 auf. Damit wird eine Differenzdruckmessung möglich.

Das anmeldungsgemäße Verschlusssystem arbeitet nunmehr nach folgendem Funktionsprinzip.

Die Pumpeneinrichtung 7 bedient sich aus dem Reservoir 5 als Speisevorrat und erzeugt einen Schließdruck am Schließelement 1, welcher derart gewählt ist, dass der Schließdruck das Körperorgan über das Schließelement abdichtet, jedoch die Durchblutung des Körperorgans nicht beeinträchtigt. Der Schließdruck entspricht einem Druckbereich, der von unterschiedlichen Parametern, wie z. B. Körperorgan, Gefäßdicke, Durchblutungsstärke usw. abhängig ist.

Bei einem kurzzeitig auftretenden Druckanstieg in dem Körperorgan, beispielsweise bei der Urethra in der Harnblase durch Husten, Niesen, Lachen oder anderwertige Anstrengung, reicht der Schließdruck nicht aus und der Durchfluss im Körperorgan würde kurzzeitig stattfinden. Für diesen Fall ist es von Vorteil, wenn der Schließdruck sich ebenfalls kurzzeitig erhöht, um somit weiterhin die Dichtheit des Körperorgans aufrecht zu erhalten. Das mit dem anmeldungsgemäßen Verschlusssystem hervorgerufene, kurzzeitige Erhöhen des Schließdruckes führt aber nicht zu einer Nekrosenbildung, d.h. die Durchblutung des Körperorgans wird nur kurzzeitig beeinflusst und ist somit für das Körperorgan nicht schädigend. Um diese Mechanik aufrechtzuerhalten, ist es notwendig, dass das Regelungssystem 3 unterschiedliche Druckbereiche aufweist. In der in Figur 1A dargestellten Ausführungsform erzeugt die Pumpeneinrichtung 7, welche beispielsweise als schnellschaltende Pumpe oder auch als schneller Aktor ausgestaltet sei, über das erste Absperrventil V1 den erforderlichen Schließdruck. Hierzu muss das zweite Absperrventil V2 geschlossen sein. Bei Erreichen des Schließdruckes wird das erste Absperrventil V1 geschlossen, wobei die Pumpeneinrichtung 7 bis zum ersten Absperrventil V1 einen gegenüber dem Schließdruck erhöhten Arbeitsdruck aufbaut. Sollte mit dem Schließelement der Durchfluss durch das Körperorgan durchgeführt werden, so muss lediglich das zweite Absperrventil V2 geöffnet werden, um den Schließdruck über das Reservoir 5 abzubauen. Treten aber nunmehr stromaufwärts des Schließelementes 1 Druckspitzen bzw. kurzzeitig anhaltende Druckbelastungen auf, so werden die mit der Sensoreinrichtung S1 registriert bzw. erfasst und an die Steuereinheit 11 weitergegeben. Die Steuereinheit 11 öffnet nahezu gleichzeitig das erste Absperrventil V1, so dass der erhöhte Arbeitsdruck das Schließelement 1 beaufschlagt und somit kurzzeitig gewährleistet, dass auch das Körperorgan gegenüber den Druckspitzen abgedichtet ist. Hierbei ist es denkbar, dass in Abhängigkeit der Durchblutungsfunktion des Körperorgans die Steuereinheit 11 nach einer Zeitkonstante das erste Absperrventil wiederum schließt und das zweite Absperrventil öffnet, um von dem erhöhten Arbeitsdruck auf den erforderlichen Schließdruck herunterzuregeln. Gleichzeitig oder zwischenzeitlich kann dann erneut die Pumpeneinrichtung 7 einen erhöhten Arbeitsdruck, welches an den erstem Arbeitsventil V1 anliegt, aufbauen und sich somit für einen zweiten Vorgang vorbereiten.

Auf diese Weise ist somit anmeldungsgemäß das Regelungssystem 3 in der Lage, von einem ersten Zustand des Verschlusssystems, welcher herkömmlicherweise mit dem Verschließen des Körperorgans über das Schließelement 1 über das wahlweise Öffnen zu vergleichen ist, eine Abweichung von dem ersten zustand selbstregulierend auf den ersten Zustand zurückzuführen. Mit dieser Maßnahme wird erreicht, dass das Verschlusssystem in jeder Situation, also auch bei Belastungen wie Husten oder Niesen, die an das Verschlusssystem gestellte Aufgabe erfüllt. Da der Schließdruck mittels einer feinsensorischen Steuerung den in dem Körperorgan stromaufwärts herrschenden Druck bzw. Blasendruck in der Harnblase angepasst wird, werden Nekroseerscheinungen verhindert, die normalerweise durch einen konstant hohen Schließdruck hervorgerufen werden, wodurch die Durchblutung des Körperorgans unterdrückt wird und somit Langzeitschäden hervorruft.

An dieser Stelle soll erneut hervorgehoben werden, dass wie in Figur 1A gezeigt ist, die Pumpeneinrichtung 7 auch über eine normale Pumpe im Zusammenspiel mit einem zweiten Reservoir R2 und einem dritten Absperrventil V3 ersetzt werden kann. In diesem Fall wird der gegenüber dem Schließdruck erhöhte Arbeitsdruck dadurch erreicht, dass die Pumpe bei geöffnetem dritten Absperrventil das Reservoir R2 bis zu dem erforderlichen Arbeitsdruck beaufschlagt, danach das dritte Absperrventil V3 dann geschlossen wird. Mit dieser Maßnahme wird erreicht, dass die Verwendung einer schnellschaltenden Pumpe nicht erforderlich ist, da die kurzzeitige Beaufschlagung des Schließelements 1 bei Auftreten von Druckspitzen über dem in dem zweiten Reservoir R2 aufgebaute Arbeitsdruck durch Öffnen des ersten Absperrventils an das Schließelement angelegt wird.

Grundsätzlich soll festgehalten werden, dass die Sensorelemente nicht lediglich als Drucksensoren betrachtet werden, sondern ebenfalls mit kapazitiven oder induktiven Sensoren der Druck bestimmt werden kann, wobei ebenso Volumenänderungen über Ultraschall oder über Dehnstreifen gemessen werden können, oder eine Abstandsänderung, die sich bei Druckerhöhung ergibt, mit Licht gemessen wird.

Die Erfindung betrifft ein System zur elektronischen Steuerung eines artifizellen feinsensorischen Sphinkterimplantats. Durch diese Erfindung soll das um eine Feinsensorik und eine Aktorik erweiterte bisherige System eines artifiziellen Sphinkterimplantats derart gesteuert werden, dass sowohl vollständige Kontinenz gewährleistet werden kann und zugleich das Risiko einer durch überhöhten und zu lange anliegenden Druck auf die natürliche Urethra erzeugten Nekrose minimiert wird.

Voraussetzung hierfür ist eine intelligente elektronische Steuerung, die verschiedene Belastungssituationen erkennt und durch Erhöhung des Cuffdruckes Inkontinenz bei dynamischer Belastung wie beispielsweise Husten oder Lachen vermeidet und zugleich durch entsprechende Drucksenkungen in Ruhe die ausreichende Durchblutung des Urethragewebes gewährleistet. Da diese elektronischen Schaltungen in einem medizinischen Implantat zum Einsatz kommen, müssen sie gewissen Anforderungen wie Zuverlässigkeit über lange Zeiträume, minimaler Stromverbrauch, kleine physikalischen Ausmaße und individuelle Anpassungsfähigkeit genügen.

Hieraus ergibt sich unmittelbar die weitere Aufgabe der Erfindung.

Erfindungsgemäß wird ferner ein System zur elektronischen Steuerung eines artifizellen feinsensorischen Sphinkterim-plantats bereitgestellt.

Weitere vorteilhafte und/oder bevorzugte Ausführungsformen der Erfindung sind Gegenstand der Unteransprüche.

Im folgenden wird dieser Aspekt der Erfindung lediglich beispielhaft und ohne Beschränkung und unter Bezugnahme auf die Figuren sowie vorteilhafte und/oder bevorzugte Ausführungsformen detaillierter beschrieben.

Die Bedeutungen der verwendeten Bezugszeichen sind in der folgenden Aufstellung angegeben. Die Begriffe Signal und Wert werden hier synonym verwendet.

(1) Differenzdrucksignal, Druckdifferenz Blase-Cuff
(2) Monostabiles Schwellensignal, Sicherheitsschwelle
(3) Bistabiles Schwellensignal, Normaldruckschwelle
(4) Cuffdruck
(5) Monostabiles Referenzsignal, Integrations-Nulloffset
(6) Monostabiles Schwellensignal, Integrationsschwelle
(7) Vordrucksignal
(8) Monostabiles Schwellensignal, Vordruckschwelle
(9) Dynamische Belastung
(10) Monostabiles Schwellensignal, Druckausgleichschwelle
(11) Blaseninnendruck
(12) Ausgangssignal bzw. Ausgangswert der Addiererfunktion, unterer Schwellenwert
(13) Ausgangssignal bzw. Ausgangswert der Integratorfunktion
(14) Ausgangssignal bzw. Ausgangswert der Differentiatorfunktion
(15) Erhöhung des Cuffdruckes
(16) Senkung des Cuffdruckes
(17) Vordruck
(18) Erhöhung des Vordruckes
(19) Ausgangssignal der Integratörfunktion des Verzögerungs-Blockes
(20) Dynamische Belastung

(21) Bereich rücklaufender Integration

(22) Bereich fortlaufender Integration

(23) Verfahren zur Regulation des Urethra-Verschlussdruckes, mittels einem aufpumpbaren Cuff, einem Fluid-Reservoir, einer Pumpe und einem Ventil über hydraulische Verbindungen

(24) Hydraulische Verbindung

(25) Cuff

(26) Steuerbares Ventil

(27) Bidirektionale Pumpe

(28) Fluid-Reservoir

(29) Vordruck-Behälter

(30) Verfahren zur Regulation des Urethra-Verschlussdruckes, mittels einem aufpumpbaren Cuff, einem Fluid-Reservoir, einer Pumpe, drei Ventilen und einem Vordruck-Behälter über hydraulische Verbindungen

(31) Urethraverschlussdruck

(32) Differentiatorfunktion

(33) Addiererfunktion

(34) Integratorfunktion

(35) Parametervariation

(36) Komparatorglied

(37) Integratorfunktion des Verzögerungsblocks

Von den Figuren zeigen

Fig. 1B: ein schematisches Funktions-Blockdiagramm ohne Vordruck-Behälter und mit dem Ausgangssignal der Differentiatorfunktion als Eingangssignal der Integratorfunktion,

Fig. 2: ein schematisches Funktions-Blockdiagramm ohne Vordruck-Behälter und mit dem Ausgangssignal der Addiererfunktion als Eingangssignal der Integratorfunktion,

Fig. 3: ein schematisches Funktions-Blockdiagramm mit Vordruck-Behälter und mit dem Ausgangssignal der Differentiatorfunktion als Eingangssignal der Integratorfunktion,

Fig. 4: ein schematisches Funktions-Blockdiagramm mit Vordruck-Behälter und mit dem Ausgangssignal der Addiererfunktion als Eingangssignal der Integratorfunktion,

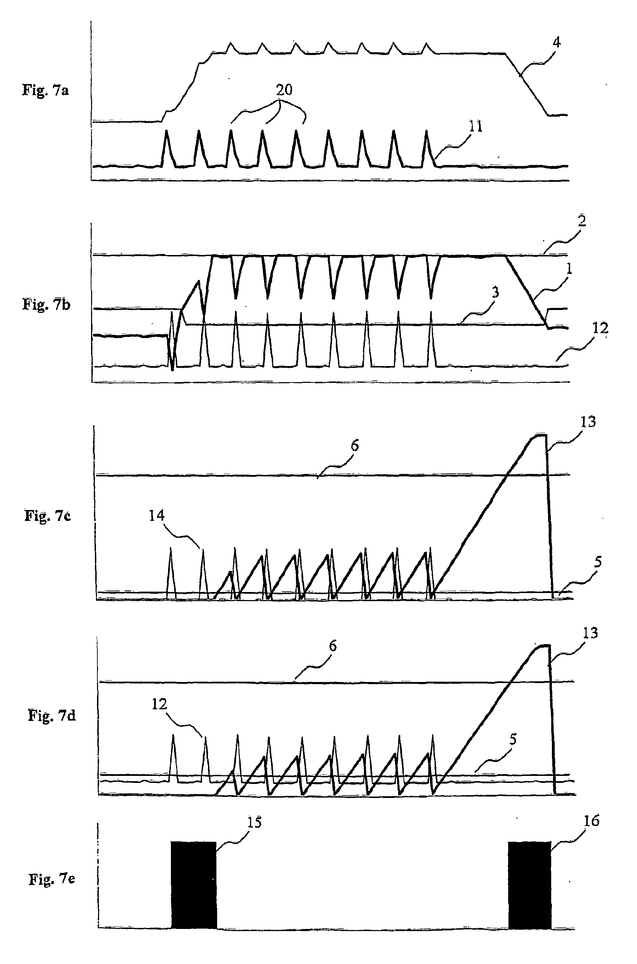

Fig. 5a = Fig. 7a: bei andauernder dynamischer Belastung den Druckverlauf im Cuff und in der Harnblase bei kurzzeitiger dynamischer Belastung,

Fig. 5b = Fig. 7b: bei andauernder dynamischer Belastung den Signalverlauf der elektronischen Schaltung bei kurzzeitiger dynamischer Belastung,

Fig. 5c = Fig. 7c: bei andauernder dynamischer Belastung den Signalverlauf des Integratorsystems mit dem Ausgangssignal der Differentiatorfunktion als Eingangssignal der Integratorfunktion bei kurzzeitiger dynamischer Belastung,

Fig. 5d = Fig. 7d: bei andauernder dynamischer Belastung den Signalverlauf des Integratorsystems mit dem Ausgangssignal der Addiererfunktion als Eingangssignal der Integratorfunktion bei kurzzeitiger dynamischer Belastung,

Fig. 5e = Fig. 7e: bei andauernder dynamischer Belastung die Schaltzustände zur Druckerhöhung und Drucksenkung im Cuff bei kurzzeitiger dynamischer Belastung,

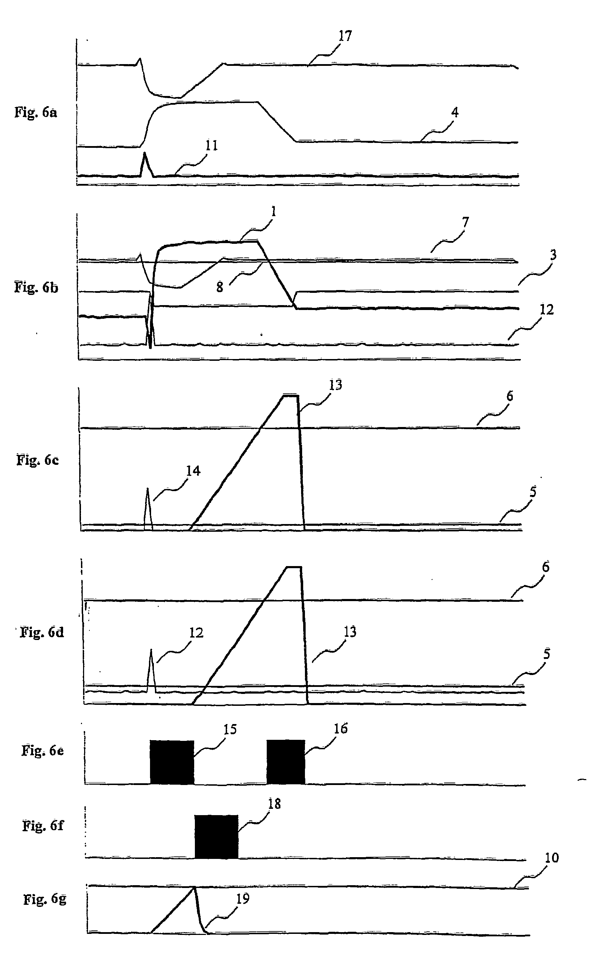

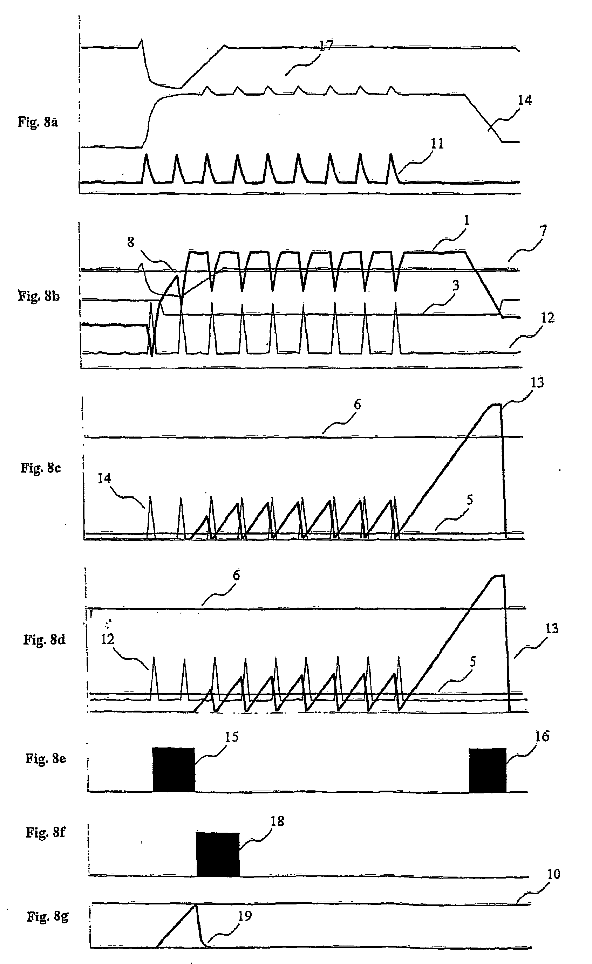

Fig. 6a = Fig. 8a: bei andauernder dynamischer Belastung den Druckverlauf im Cuff, in der Harnblase und dem Vordruckbehälter bei kurzzeitiger dynamischer Belastung,

Fig. 6b = Fig. 8b: bei andauernder dynamischer Belastung den Signalverlauf der elektronischen Schaltung bei kurzzeitiger dynamischer Belastung,

Fig. 6c = 8c: bei andauernder dynamischer Belastung den Signalverlauf des Integratorsystems mit dem Ausgangssignal der Differentiatorfunktion als Eingangssignal der Integratorfunktion bei kurzzeitiger dynamischer Belastung,

Fig. 6d = Fig. 8d: bei andauernder dynamischer Belastung den Signalverlauf des Integratorsystems mit dem Ausgangssignal der Addiererfunktion als Eingangssignal der Integratorfunktion bei kurzzeitiger dynamischer Belastung,

Fig. 6e = Fig. 8e: bei andauernder dynamischer Belastung die Schaltzustände der Druckerhöhung und Drucksenkung im Cuff bei kurzzeitiger dynamischer Belastung,

Fig. 6f = Fig. 8f: bei andauernder dynamischer Belastung die Schaltzustände der Druckerhöhung im Vordruck-Behälter,

Fig. 6g = Fig. 8g: bei andauernder dynamischer Belastung den Verlauf des Integratorsignals zur Steuerung der Öffnung des Ventils zwischen Cuff und Vordruck-Behälter,

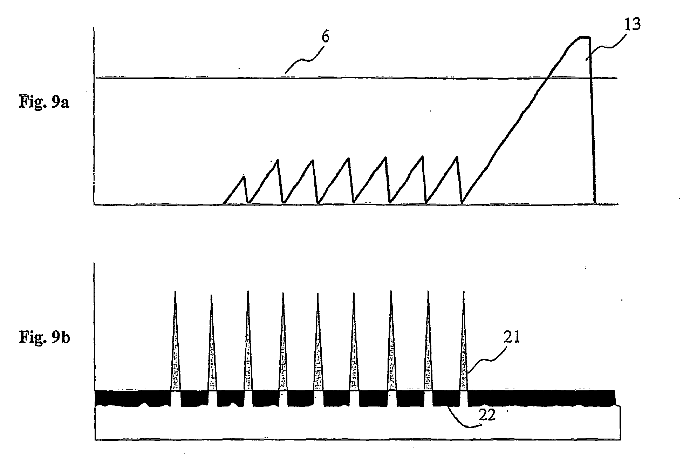

Fig. 9a: das Integratorsignal mit Schaltschwelle bei andauernder dynamischer Belastung,

Fig. 9b: die Eingangssignale der Integratorfunktion mit Bereichen fortlaufender (22) und rücklaufender (21) Integration bei andauernder dynamischer Belastung,

Fig. 10: das Verfahren zur Regulation des Urethra-Verschlussdruckes ohne Vordruck-Behälter,

Fig. 11: das Verfahren zur Regulation des Urethra-Verschlussdruckes mit Vordruck-Behälter,

Fig. 12 den Signalverlauf bei dynamischer Belastung, und zwar 12a Absolutdrücke, 12b das Urethraverschlussdrucksignal mit Schaltschwellen und 12c das Integratorsignal mit Schaltschwellen (bzw. Integratorsystem bzw. Integratorelement), und

Fig. 13 eine funktionelle Prinzipdarstellung des artifiziellen Sphinkterimplantats.

Die elektronische Steuerung des Sphinkterimplantats kann so-wohl digital als auch analog realisiert werden. Das Verfahren zur Steuerung des Sphinkterimplantats, das Gegenstand dieser Erfindung ist, ändert sich prinzipiell damit nicht.

Dem Patienten wird mithilfe variierbarer Parameter die Möglichkeit gegeben, das verhalten des Implantats individuell an die persönlichen Anforderungen anzupassen. Bei einer analogen Steuerung werden zur Parametervariation D/A-Wandler verwendet, deren Taktsignal extern erzeugt und über die Telemetrie übertragen werden kann.

Funktionsweise der elektronischen Steuerung

Die elektronische Steuerung, die Gegenstand dieser Erfindung ist, setzt Signale der Feinsensorik, insbesondere des Differenzdrucks zwischen Harnblase und Cuff oder eines vergleichbaren Differenzdrucks, in Steuerbefehle an die Aktorik des Implantats um.

Die an den Signaleingängen angelegten Sensorsignale werden mittels Verstärkerschaltungen derart verstärkt, dass sich die Signale im Betrieb zwischen den durch die Schaltung vorgegebenen Grenzwerten bewegen.

Im Betrieb ohne dynamische Belastung, etwa beim ruhigen Sitzen oder Liegen, bewegt sich der Cuffdruck (4) in einem Bereich, in dem die Durchblutung des betreffenden Urethragewebes gewährleistet ist, bei Erhöhung des Blaseninnendrucks (11) aber leicht zur Inkontinenz führen könnte. Steigt das Differenzdrucksignal bzw. der Differenzdruckwert (1), wird bei Erreichen eines bistabilen Schwellenwerts (3) der Cuffdruck (4) gesenkt (16), unterschreitet das Differenzdrucksignal bzw. der Differenzdruckwert (1) den abgesenkten bistabilen Schwellenwert (3), wird die Senkung (16) des Cuffdruckes (4) beendet. Dies geschieht bei der analogen Variante mit Hilfe einer Komparatorfunktion, die mit einer Hysterese-Schaltung versehen ist.

Bei Auftreten dynamischer Belastung muss die Aktorik die Funktion eines gesunden Sphinkters übernehmen, nämlich durch aktive Drucktransmission, also die unwillkürliche Kontraktion des Sphinkters bei dynamischer Belastung Inkontinenz vermeiden. Bei plötzlicher Druckerhöhung, etwa beim Husten oder Lachen, muss innerhalb von Millisekunden der Cuffdruck (4) erhöht werden. Da bei einem Differenzdruck von Null von Inkontinenz ausgegangen werden kann, wird bei Unterschreiten des Differenzdrucksignals bzw. des Differenzdruckwertes (1) unter einen von Null verschiedenen Schwellenwert die Aktorik zu Erhöhung (15) des Cuffdruckes (4) veranlasst. Um einer gewissen Trägheit der Aktorik darüber hinaus vorzubeugen, ist in der elektronischen Steuerung vorgesehen, diesen unteren Schwellenwert, den das Differenzdrucksignal bzw. der Differenzdruckwert (1) nur bei Miktion unterschreiten darf, mit einer additiven, aktiven Komponente auszustatten. Um dies zu erreichen, wird bei der analogen Variante das Differenzdrucksignal (1) mithilfe einer Differentiatorschaltung differenziert und das Ausgangssignal bzw. den Ausgangswert (14) der Differentiatorfunktion mithilfe einer Addiererschaltung um den unteren Schwellenwert angehoben. Bei Einsatz eines Mikroprozessors wird das Sensorsignal numerisch differenziert, indem vom vorletzten Signalwert der aktuelle Signalwert subtrahiert wird und das Ergebnis, falls positiv, mit einer Offset-Komponente addiert wird. Das Ergebnis dient als unterer Schwellenwert (12). Durch diese Maßnahmen wird erreicht, dass der untere Schwellenwert (12) bei plötzlicher Erhöhung des Blaseninnendruckes (11) dem sinkenden Differenzdrucksignal bzw. dem Differenzdruckwert (1) entgegenläuft und so ein frühzeitiges Aktivieren der Aktorik ermöglicht. Die Aktivierung der Aktorik erfolgt bei der analogen Variante mittels eines Komparators, der das Differenzdrucksignal (1) mit dem unteren Schwellenwert (12) vergleicht.

Sobald durch Unterschreiten des Differenzdrucksignals (1) unter den unteren Schwellenwert (12) die Aktorik aktiviert wird, wird bei der analogen Variante die Komparatorfunktion zur Vermeidung eines überhöhten Cuffdruckes (4) im unbelasteten Betrieb deaktiviert und der Cuffdruck (4) auf einen Wert erhöht, bei dem Kontinenz gewährleistet werden kann, die Durchblutung des betreffenden Urethragewebes aber beeinträchtigt sein kann.

Diese Druckerhöhung kann, je nach Auslegung der Aktorik, auf unterschiedliche Weisen erfolgen:

Beim Einsatz eines Vordruck-Behälters (29) wird ein Ventil (26) geöffnet, so dass ein Druckausgleich zwischen dem Vordruck-Behälter (29) und dem Cuff (25) stattfinden kann. Aufgrund des Fluidflusses bei diesem Druckausgleich muss der vorher eingestellte Vordruck (17) je nach Konstruktion des Cuffs (25) und des Vordruck-Behälters (29) um einen bestimmten Betrag höher sein als der gewünschte maximale Cuffdruck. Das Öffnen des Ventils zwischen Vordruck-Behälter (29) und Cuff (25) erfolgt zeitgesteuert. Dabei kann bei der analogen Variante insbesondere ein Integratorglied zum Einsatz kommen, das

gleichzeitig mit dem Öffnen des Ventils gestartet wird und dessen Ausgangssignal (19) mittels eines Komparatorgliedes mit einem monostabilen Schwellenwert (10) verglichen wird. Bei Erreichen der Parität wird das Ventil (26) zwischen Vordruck-Behälter (29) und Cuff (25) wieder geschlossen und gleichzeitig die Erhöhung (18) des Vordruckes (17) im Vordruck-Behälter (29) gestartet. Die Aktivierung des Integratorgliedes, das bei der analogen Variante die Senkung (16) des Cuffdruckes (25) steuert, erfolgt entweder gleichzeitig mit dem Öffnen oder mit dem Schließen des Ventils (26) zwischen Vordruck-Behälter (29) und Cuff (25). Das Signal, das das Ventil (26) zwischen Vordruck-Behälter (29) und Cuff (25) zum Schließen veranlasst, löst die Erhöhung (18) des Vordrukkes (17) im Vordruck-Behälter (29) aus. Das Druck-Signal des Vordruckes (17), das beim Einsatz eines Vordruck-Behälters (29) zusätzlich benötigt wird, wird bei der analogen Variante mittels eines Komparatorgliedes mit einem monostabilen Schwellenwert (8) verglichen und bei Parität wird ein Signal ausgelöst, welches die Beendigung der Erhöhung (18) des Vordruckes (17) veranlasst. Bei Einsatz eines Mikroprozessors wird dieser so programmiert, dass er sich prinzipiell wie die beschriebene analoge Schaltung verhält.

Bei anders ausgelegter Aktorik ohne Vordruck-Behälter (29) wird das Erreichen dieses Sicherheitsdruckes mit einem Komparatorglied bzw. einer Komparatorfunktion registriert, das das Differenzdrucksignal bzw, den Differenzdruckwert (1) mit einem monostabilen Schwellenwert (2) vergleicht und bei Überschreiten des Differenzdrucksignals bzw. des Differenzdruckwertes (1) über diesen Schwellenwert (2) ein Signal auslöst, das die Aktorik zur Beendigung der Erhöhung (15) des Cuffdruckes (4) veranlasst. Durch dieses Signal wird außerdem das Integratorglied bzw. die Integratorfunktion aktiviert, das die Senkung (16) des Cuffdruckes (4) steuert.

Die Aktivierung des Integratorgliedes, das bei der analogen Variante die Senkung (16) des Cuffdruckes (4) steuert, kann insbesondere über die Unterbrechung eines Entlade-Stromkreises des dem Operationsverstärker des Integratorgliedes gegengekoppelten Kondensators erfolgen. Somit kann eine Integration stattfinden und Startwert ist gleich der Spannung über dem entladenen Kondensator.

Das intelligente Verhalten der elektronischen Steuerung zeigt sich insbesondere darin, dass bei andauernder dynamischer Belastung die Senkung (16) des Cuffdruckes (4) verzögert wird. Bei der analogen Variante kommt hierzu ein Integratorglied zum Einsatz. Durch eine geeignete Wahl der Eingangssignale wird erreicht, dass bei kurzzeitiger Belastung, beispielsweise beim Aufstehen aus sitzender Position, der Cuffdruck (4) recht schnell wieder auf Werte gesenkt wird, die dem sicheren Normalbetrieb ohne Nekroserisiko entsprechen. Bei andauernder dynamischer Belastung hingegen, beispielsweise bei körperlichen Tätigkeiten, wird der Cuffdruck (4) solange auf dem hohen Niveau gehalten, bis die dynamische Belastung nachlässt. Dieses Verhalten wird bei der analogen Variante dadurch erzeugt, dass am invertierenden und am nicht invertierenden Eingang des Operationsverstärkers des Integratorgliedes zwei geeignete Signale anliegen. Zum einen ist das ein dem Ausgangssignal (14) des Differentatorgliedes analoges Signal, insbesondere entweder das Ausgangssignal (14) des Differentiatorgliedes selbst oder das Ausgangssignal (12) des Addierergliedes. Am anderen Eingang des Operationsverstärkers des Integratorgliedes liegt ein monostabiles Referenzsignal (5). Dieses monostabile Referenzsignal (5) ist derart gewählt, dass es von dem am anderen Eingang des Operationsverstärkers des Integratorgliedes anliegenden Signal bei dynamischer Belastung und damit volatilen Ausgangssignal (14) des Differentatorgliedes gekreuzt werden kann. Diese Beschaltung des Integratorgliedes hat zur Folge, dass das Ausgangssignal (13) des Integratorgliedes sich vom Startpunkt entfernt, wenn das dem Ausgangssignal (14) des Differentiatorgliedes analoge Eingangssignal das monostabile Referenzsignal (5) nicht oder selten gekreuzt hat, also bei geringer Dynamik der Belastung. Ist die Dynamik der Belastung dagegen hoch, kreuzt das dem Ausgangssignal (14) des Differentiatorgliedes analoge Eingangssignal das monostabile Referenzsignal (5) häufig. Sobald das dem Ausgangssignal (14) des Differentiatorgliedes analoge Eingangssignal das monostabile Referenzsignal (5) gekreuzt hat, bewegt sich das Ausgangssignal (13) des Integratorgliedes auf den Startwert zu. Bei hoher Dynamik der Belastung wird somit das Ausgangssignal (13) des Integratorgliedes Zickzack-förmig gegen den Startwert getrieben. Bei der analogen Variante wird durch ein Komparatorglied das Ausgangssignal (13) des Integratorgliedes mit einem konstanten Schwellenwert (6), der nicht in der Nähe des Startwertes des Ausgangssignals (13) des Integratorgliedes liegen darf, verglichen. Sobald das Ausgangssignal (13) des Integratorgliedes diesen konstanten Schwellenwert (6) erreicht, wird die Senkung (16) des Cuffdruckes (4) veranlasst. Diese Senkung erfolgt durch die Aktivierung des Komparatorgliedes, welches die Aktorik solange zur Senkung (16) des Cuffdruckes (4) veranlasst, bis das Differenzdrucksignal (1) das abgesenkte bistabile Schwellensignal (3) unterschreitet. Der Cuffdruck (4) liegt nun wieder in einem sicheren Bereich, bei dem die Nekrosegefahr minimal ist. Kommt ein Mikroprozessor zum Einsatz, führt dieser numerisch prinzipiell die gleichen Berechnungen durch, wie die analoge Schaltung und erzeugt damit ein vergleichbares Verhalten.

Eine weitere entscheidende Eigenschaft dieser elektronischen Steuerung ist das Vorhandensein zahlreicher Möglichkeiten, das Verhalten des Systems durch die Variation von Parametern von außen zu beeinflussen. Dadurch kann das artifizielle feinsensorische Sphinkterimplantat an die individuellen Anforderungen des Patienten angepasst werden, zum Einen die Einstellung der Parameter nach Vollendeter Vernarbung nach der Implantation, zum Anderen bei sich ändernden Anforderungen durch den Patienten, beispielsweise bei fortschreitendem Alter oder der Änderung der Lebensgewohnheiten.

Da man bei der analogen Variante im Gegensatz zur digitalen die Anzahl der variierbaren Parameter begrenzen muß, sind zur Variation vier Parameter ausgewählt worden, die das Verhalten des Implantats entscheidend beeinflussen können.

Das monostabile Offsetsignal

Das monostabile Offsetsignal ist die additive Komponente, die das Augangssignal (14) des Differentiatorgliedes zur unteren Schaltschwelle ergänzt. Durch Variation des monostabilen Offsetsignals kann eine zu träge Aktorik oder ein vorzeitiger Urinfluß ausgeglichen werden.

Das bistabile Schwellensignal (3)

Das bistabile Schwellensignal (3) kann variiert werden, wenn der Cuffdruck (4) im Normalbetrieb schon für die Durchblutung des Urethragewebes kritische Werte errecht, oder wenn das bistabile Schwellensignal (3) derart niedrig eingestellt ist, dass das Absenken (16) des Cuffdruckes (4) unangemessen häufig veranlasst wird.

Das monostabile Referenzsignal (5)

Durch Variation des monostabilen Referenzsignals (5) kann die Geschwindigkeit der Integration angepasst werden.

Das monostabile Schwellensignal (2)

Das monostabile Schwellensignal (2) entspricht dem Differenzdrucksignal bzw. dem Differenzdruckwert (1), bei dem der Cuffdruck (4) einen Wert hat, bei dem Kontinenz gewährleistet, die Durchblutung des Urethragewebes aber beeinträchtigt ist. Wenn das monostabile Schwellensignal (2) zu niedrig liegt, kann trotz erhöhtem Cuffdruck (4) bei starker dynamischer Belastung das Differenzdrucksignal bzw. der Differenzdruckwerk (1) unter die untere Schaltschwelle fallen und so eine weitere Duckerhöhung auslösen. Bei einem zu hoch eingestellten monostabilen Schwellensignal (2), kann das Urethragewebe infolge des zu hohen Cuffdruckes (4) Schaden nehmen

oder

Das monostabile Schwellensignal (8)

Durch Variation des monostabilen Schwellensignals (8) wird der Vordruck im Vordruckbehälter und somit der maximale Cuffdruck (4) nach dem Druckausgleich eingestellt.

Kommt ein Mikroprozessor zum Einsatz, hat man von vornherein wesentlich mehr Möglichkeiten, das Verhalten zu beeinflussen, bis hin zur vollständigen Neuprogrammierung.

Die Verfahren zur Regulation_des_Cuffdruckes

Die von der beschriebenen analogen Schaltung gesteuerte Aktorik kann sich verschiedener Verfahren und Anordnungen bedienen, um den gewünschten Einfluß auf die Urethra zu erzeugen. Die unterschiedlichen Anordnungen und Komponenten der Aktorik erfordern eine angepasste Steuerelektronik. Nachfolgend werden beispielhaft zwei hydraulische Verfahren beschrieben, eines mit und eines ohne Vordruck-Behälter (29).

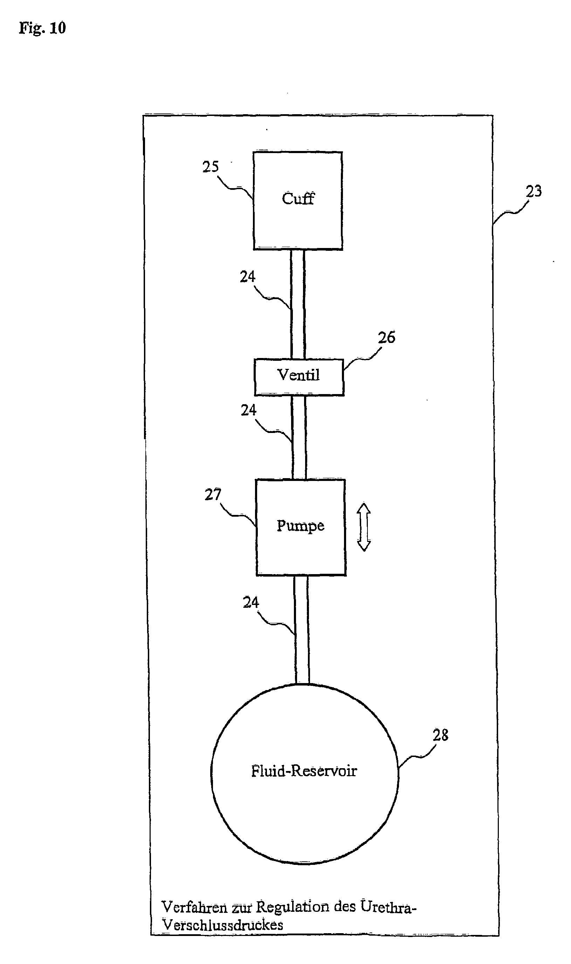

Bezüglich des Verfahrens zur Regulation des Cuffdruckes (4) mittels einer Pumpe (27), einem Ventil (26) und einem Fluid-Reservoir (28) über hydraulische Verbindungen (24), siehe Fig. 10.

Bei diesem Verfahren zur Regulation des Cuffdruckes (4) ist der Einsatz einer Pumpe (27) vorgesehen, die bei Stillstand dem hydraulischen Fluß einen geringen Widerstand entgegensetzt. Bei Öffnen des Ventils (26) zur Senkung (16) des Cuffdruckes (4) findet zwischen Cuff (25) und Fluid-Reservoir (28) ein Druckausgleich statt. Das Ventil (26) kann sowohl zwischen Cuff (25) und Pumpe (27) als auch zwischen Pumpe (27) und Fluid-Reservoir (28) platziert werden.

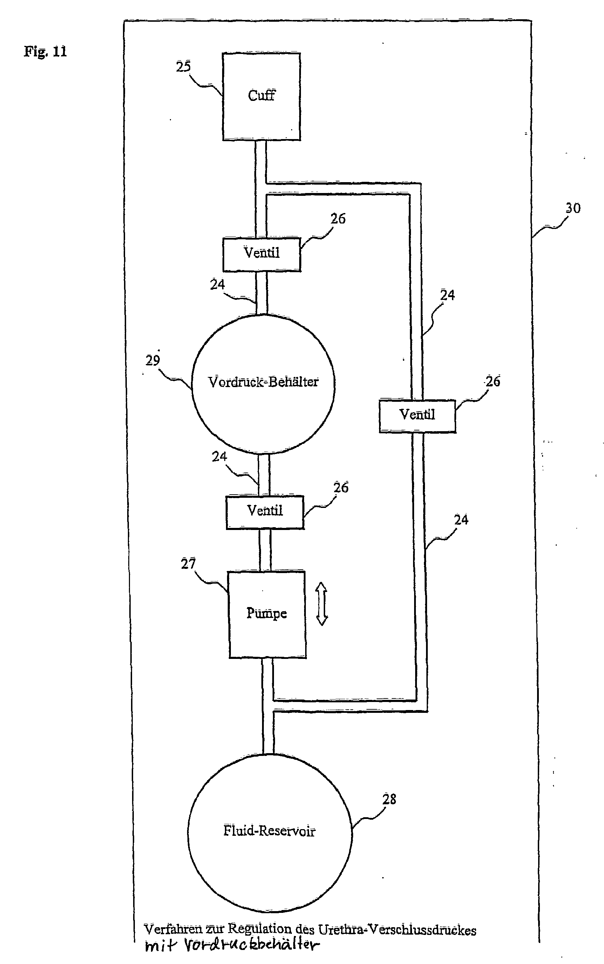

Bezüglich des Verfahrens zur Regulation des Cuffdruckes (4) mittels einer Pumpe (27), einem Vordruck-Behälter (29), drei Ventilen (26) und einem Fluid-Reservoir (28) über hydraulische Verbindungen (24), siehe Fig. 11.

Der Einsatz eines Vordruck-Behälters (29) bei diesem Verfahren ermöglicht eine blitzartige Erhöhung (15) des Cuffdruckes (4). Allerdings wird ein weiterer Drucksensor benötigt, der die Regulation des Vordrukkes (17) steuert. Nach Auslösen der Erhöhung (15) des Cuffdruckes (4) wird durch Öffnen des Ventils (26) zwischen Vordruck-Behälter (29) und Cuff (25) ein Druckausgleich ermöglicht. Der Sicherheitsdruck im Cuff (25) hängt also nur von dem zuvor im Vordruck-Behälter (29) eingestellten Vordruck (17) ab. Entscheidend für den Druckausgleich ist auch die Konstruktion des Vordruck-Behälters (29). Je kleiner die Ausmaße, umso größer muss der Vordruck (17) sein.

Da zur Erhöhung (18) des Vordruckes (17) der Zeitraum nach Erhöhung (15) des Cuffdruckes (4) genutzt wird, also für einen gewissen Zeitraum keine weitere Erhöhung (15) des Cuffdruckes (4) ausgelöst werden kann, muß bei der Auswahl der Pumpe (27) keine hohen Anforderungen an Geschwindigkeit gelegt werden. Der Betrieb an geringen Spannungen wird dadurch ermöglicht.

Die Miktionskontrolle

Zur Einleitung der Miktion wird extern ein Signal erzeugt. Dieses Signal veranlasst die Aktorik zur Senkung (16) des Cuffdruckes (4) und deaktiviert darüber hinaus die analoge elektronische Schaltung bis auf das Komparatorglied, das den Cuffnormaldruck nach oben begrenzt.

Nach Beendigung der Miktion wird ein zweites externes Signal erzeugt, das wieder die Erhöhung (15) des Cuffdruckes (4) einleitet. Der Cuffdruck (4) wird solange erhöht, bis das Differenzdrucksignal bzw. der Differenzdruckwert (1) den bistabilen Schwellenwert (3) errecht. Durch einen Komparator wird damit die restliche analoge elektronische Schaltung reaktiviert.

Zusammengefasst umfasst dieser Aspekt der Erfindung somit folgende Ausführungsformen:

Nach Ausführungsform 1 ist, das system zur elektronischen Steuerung eines artifiziellen feinsensorischen Sphinkterimplantats **dadurch gekennzeichnet, dass** das Verhalten mindestens eines Sensorsignals in einer analogen oder digitalen elektronischen Schaltung mittels Rechen- und Komparatorfunktionen derart umgewandelt und mit Referenzwerten verglichen wird, dass eine Aktorik derart gesteuert wird, dass sich der Cuffdruck bzw. der Differenzdruck zwischen Cuff und Harnblase entweder in einem durch zwei Schwellenwerte begrenzten niedrigen Bereich bewegt oder oberhalb eines bestimmten Sicherheitsdruckes.

Nach Ausführungs form , welche zusammen mit Ausführungsform 1 die Erfindung darstellt, ist das Sensorsignal bzw. der Sensor-wert (1) der elektronischen Schaltung nach Ausführungsform 1 **dadurch gekennzeichnet, dass** es sich analog der Differenz zwischen Blaseninnendruck (11) und Cuffdruck (4) verhält.

Nach Ausführungsform 3 ist die Differentiatorfunktion der elektronischen Schaltung nach Ausführungsform 1 **dadurch gekennzeichnet, dass** das Ausgangssignal bzw. der Ausgangswert (14) der Differentiatorfunktion der Differentiation des Verlaufes des Sensorsignals (1) nach Ausführungsform 2 entspricht.

Nach Ausführungsform 4 ist die Addiererfunktion mit einem monostabilen Offsetsignal bzw. Offsetwert der elektronischen Schaltung nach Ausführungsform 1 **dadurch gekennzeichnet, dass** das Ausgangssignal bzw. der Ausgangswert (12) der Addiererfunktion dem um ein monostabiles Offsetsignal bzw. einen monostabilen Offsetwert erhöhten Ausgangssignal bzw. Ausgangswert (14) der Differentiatorfunktion nach Ausführungsform 3 entspricht.

Nach Ausführungsform 5 ist die Komparatorfunktion mit einem bistabilen Schwellensignal bzw. Schwellenwert (3) der elektronischen Schaltung nach Ausführungsform 1 **dadurch gekennzeichnet, dass** für den Fall der Parität bzw. der Kreuzung des Sensorsignals bzw. Sensorwertes (1) nach Ausführungsform 2 mit dem bistabilen Schwellensignal bzw. dem bistabilen Schwellenwert (3) dieses Schwellensignal bzw. diesen Schwellenwert (3) um den durch die Hysterese bedingten Betrag abgesenkt bzw. angehoben wird und ein Signal ausgelöst wird, das die Aktorik zur Absenkung (16) des Cuffdruckes (4) veranlasst solange, bis durch nochmalige Parität bzw. nochmaliges Kreuzen des Sensorsignals (1) nach Ausführungsform 2 mit dem bistabilen Schwellensignal bzw. dem bistabilen Schwellenwert (3) ein Signal ausgelöst wird, das die Aktorik zur Beendigung der Absenkung (16) des Cuffdruckes (4) veranlasst und das bistabile Schwellensignal bzw. den bistabilen Schwellenwert (3) um den durch die Hysterese bedingten Betrag anhebt bzw. absenkt.

Nach Ausführungsform 6 ist die Komparatorfunktion der elektronischen Schaltung nach Ausführungsform 1 **dadurch gekennzeichnet, dass** das Sensorsignal bzw. der Sensorwert (1) nach Ausführungsform 2 mit dem Ausgangssignal bzw. dem Ausgangswert (12) der Addiererfunktion nach Ausführungsform 4 derart verglichen wird, dass für den Fall der Parität bzw. der Kreuzung des Sensorsignals bzw. des Sensorwertes (1) nach Ausführungsform 2 mit dem Ausgangssignal bzw. dem Ausgangswert (12) der Addiererfunktion nach Ausführungsform 4 ein Signal ausgelöst wird, das die Aktorik zur Erhöhung (15) des Cuffdruckes (4) veranlasst und die Komparatorfunktion nach Ausführungsform 5 außer Kraft setzt.

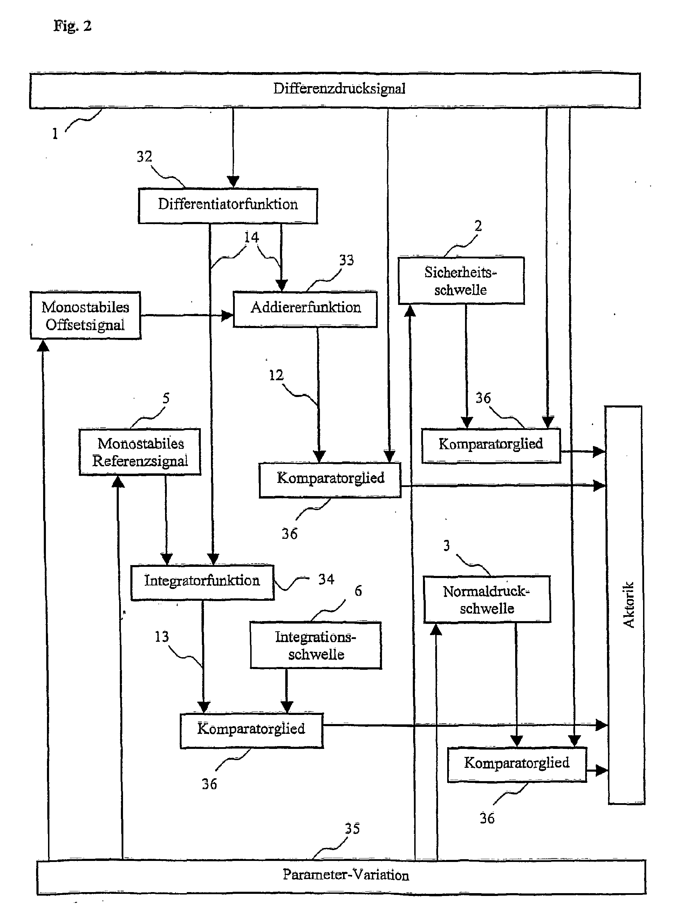

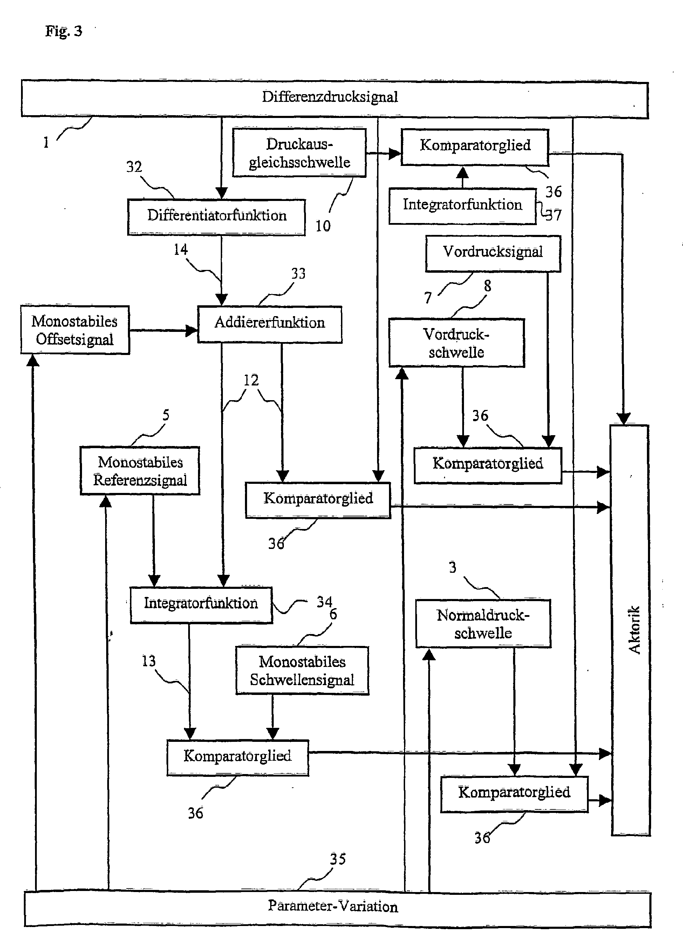

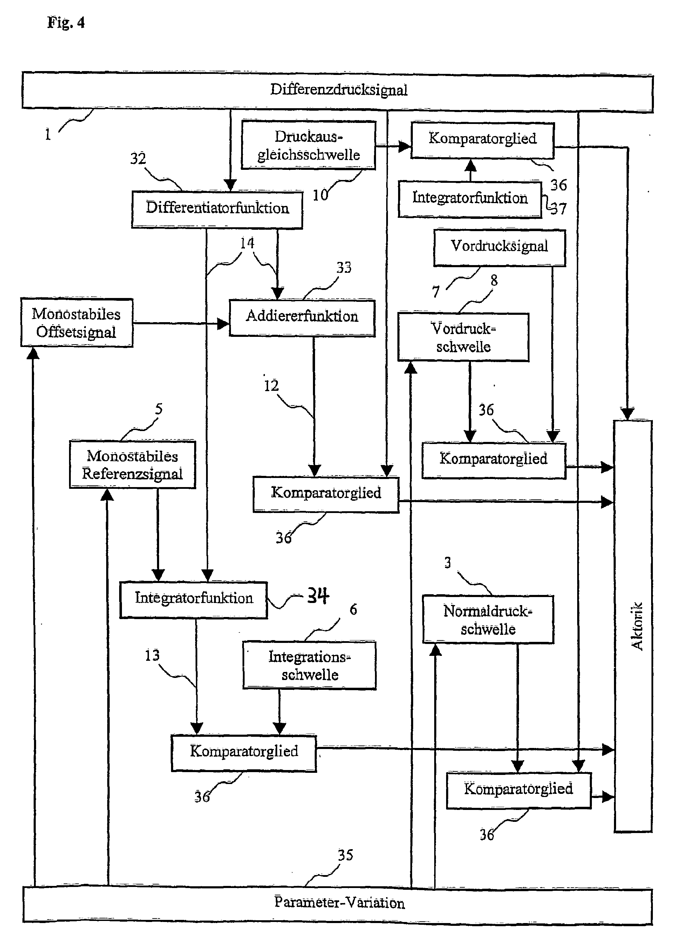

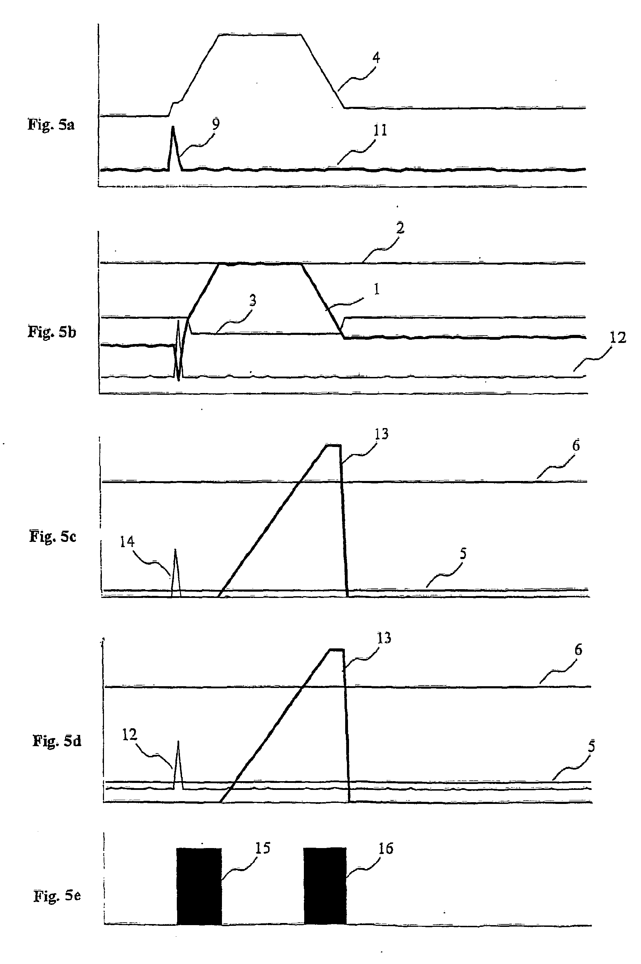

Nach Ausführungsform 7 ist die Integratorfunktion mit einem monostabilen Referenzsignal bzw. einem monostabilen Referenzwert (5) und einem konstanten Startwert der elektronischen Schaltung nach Ausführungsform 1 **dadurch gekennzeichnet, dass** wahlweise

a) das monostabile Referenzsignal bzw. der monostabile Referenzwert (5) ungleich dem monostabilen Offsetsignal bzw. Offsetwert nach Ausführungsform 4 ist und derart gewählt ist, dass das Ausgangssignal bzw. der Ausgangswert (12) der Addiererfunktion nach Ausführungsform 4 bei Aktivität das monostabile Referenzsignal bzw. den monostabilen Referenzwert (5) kreuzt, und dass die Integratorfunktion die Differenz zwischen dem Ausgangssignal bzw. dem Ausgangswert (12) der Addiererfunktion nach Ausführungsform 4 und dem monostabilen Referenzsignal bzw. dem monostabilen Referenzwert (5) derart kontinuierlich oder numerisch integriert, dass sich bei geringer Aktivität des Ausgangssignals bzw. des Ausgangswertes (14) der Differentiatorfunktion nach Ausführungsform 3 das Ausgangssignal bzw. der Ausgangswert (13) der Integratorfunktion vom Startwert entfernt und bei hoher Aktivität des Ausgangssignals bzw. des Ausgangswertes (14) der Differentiatorfunktion nach Ausführungsform 3 auf den Startwert zubewegt, siehe Fig. 1d, Fig. 2d, Fig. 3d, Fig. 4d und Fig. 5, oder

b) das monostabile Referenzsignal bzw. der monostabile Referenzwert (5) ungleich dem Ausgangssignal bzw. Ausgangswert (14) der Differentiatorfunktion nach Ausführungsform 3 bei geringer Aktivität ist und derart gewählt ist, dass das Ausgangssignal bzw. der Ausgangswert (14) der Differentiatorfunktion nach Ausführungsform 3 bei Aktivität das monostabile Referenzsignal bzw. den monostabilen Referenzwert (5) kreuzt, und dass die Integratorfunktion die Differenz zwischen dem Ausgangssignal bzw. dem Ausgangswert (14) der Differentiatorfunktion nach Ausführungsform 3 und dem monostabilen Referenzsignal bzw. dem monostabilen Referenzwert (5) derart kontinuierlich oder numerisch integriert, dass sich bei geringer Aktivität des Ausgangssignals bzw. des Ausgangswertes (14) der Differentiatorfunktion nach Ausführungsform 3 das Ausgangssignal bzw. der Ausgangswert (13) der Integratorfunktion kontinuierlich vom Startwert entfernt und bei hoher Aktivität des Ausgangssignals bzw. des Ausgangswertes (14) der Differentiatorfunktion nach Ausführungsform 3 auf den Startwert zubewegt, siehe Fig. 1c, Fig. 2c, Fig. 3c, Fig. 4c und Fig. 5.

Nach Ausführungsform 8 ist die Verzögerungsfunktion der elektronischen Schaltung nach Ausführungsform 1 bei Einsatz eines Verfahrens zur Regulation des Cuffdruckes (4) mit Vordruck-Behälter (29), **dadurch gekennzeichnet, dass** bei Auslösen des Druckausgleiches zwischen dem Vordruck-Behälter (29) und dem Cuff (25) durch die Komparatorfunktion nach Ausführungsform 6 die Verzögerungsfunktion aktiviert wird und bei Erreichen der eingestellten Verzögerungszeit ein Signal auslöst, das die Aktorik zur Beendigung des Druckausgleichs zwischen dem Vordruck-Behälter (29) und dem Cuff (25) veranlasst und die Integratorfunktion nach Ausführungsform 7 derart aktiviert, dass bei Aktivierung das Ausgangssignal bzw. der Ausgangswert (13) der Integratorfunktion nach Ausführungsform 7 dem konstanten Startwert nach Ausführungsform 7 entspricht.

Nach Ausführungsform 9 ist die Komparatorfunktion mit einem monostabilen Schwellensignal bzw. Schwellenwert (2) der elektronischen Schaltung nach Ausführungsform 1 **dadurch gekennzeichnet, dass** für den Fall der Parität bzw. der Kreuzung des Sensorsignals bzw. Sensorwertes (1) nach Ausführungsform 2 mit dem monostabilen Schwellensignal bzw. den monostabilen Schwellenwert (2) ein Signal ausgelöst wird, das die Aktorik zur Beendigung der Erhöhung (15) des Cuffdruckes (4) veranlasst und die Integratorfunktion nach Ausführungsform 7 derart aktiviert, dass bei Aktivierung das Ausgangssignal bzw. der Ausgangswert (13) der Integratorfunktion nach Ausführungsform 7 dem konstanten Startwert nach Ausführungsform 7 entspricht.

Nach Ausführungsform 10 ist die Komparatorfunktion mit einem monostabilen Schwellensignal bzw. einem monostabilen Schwellenwert (6) der elektronischen Schaltung nach Ausführungsform 1 **dadurch gekennzeichnet, dass** für den Fall der Parität bzw. der Kreuzung des Ausgangssignals bzw. des Ausgangswertes (13) der Integratorfunktion nach Ausführungsform 7 mit dem monostabilen Schwellensignal bzw. Schwellenwert (6) ein Signal ausgelöst wird, das die Aktorik zur Senkung (16) des Cuffdruckes (4) veranlasst und/oder die Komparatorfunktion nach Ausführungsform 5 aktiviert.

Nach Ausführungsform 11 ist das monostabile Offsetsignal bzw. der monostabile Offsetwert nach Ausführungsform 4 **dadurch gekennzeichnet, dass** es durch Parameter-Variation von außen variiert werden kann.

Nach Ausführungsform 12 ist das bistabile Schwellensignal bzw. der bistabile Schwellenwert (3) nach Ausführungsform 5 **dadurch gekennzeichnet, dass** es durch Parameter-Variation von außen variiert werden kann.

Nach Ausführungsform 13 ist das monostabile Referenzsignal bzw. der monostabile Referenzwert (5) nach Ausführungsform 7 **dadurch gekennzeichnet, dass** es durch Parameter-Variation von außen variiert werden kann.

Nach Ausführungsfom 14 ist das monostabile Schwellensignal bzw. der monostabile Schwellenwert (2) nach Ausführungsform 9 **dadurch gekennzeichnet, dass** es durch Parameter-Variation von außen variiert werden kann.

Nach Ausführungsform 15 ist das system zur Kontrolle der Miktion der elektronischen Schaltung nach Ausführungsform 1 **dadurch gekennzeichnet, dass** zur Aktivierung der Miktion ein externes Signal die Aktorik zur Senkung des Urethra-Verschlussdruckes veranlasst und die elektronische Schaltung nach Ausführungsform 1 bis auf die Komparatorfunktion nach Ausführungsform 5 deaktiviert und zur Deaktivierung der Miktion ein externes Signal die Aktorik zur Erhöhung (15) des Cuffdruckes (4) veranlasst bis für den Fall der Parität bzw. der Kreuzung des Sensorsignals (1) nach Ausführungsform 2 mit dem bistabilen Schwellensignal bzw. Schwellenwert (3) nach Ausführungsform 5 ein Signal ausgelöst wird, das die elektronische Schaltung nach Ausführungsform 1 aktiviert.

In dieser Erfindung wird ein neuartiges feinsensorisches Implantat vorgestellt, das die wichtigsten Aspekte technologischer Problemstellungen der in-vivo-Medizintechnik in sich vereint: Eine zuverlässige Feinsensorik, eine intelligente, flexible Steuerelektronik mit geringer Leistungsaufnahme, eine miniaturisierte und leistungsstarke Aktorik sowie eine ausgereifte Energie- und Datenübertragung. Am Beispiel dieses Implantats, eines artifiziellen, adaptiven, feinsensorischen Sphinkters, soll vor dem Hintergrund der Entwicklungsgeschichte und der methodischen Kriterienbildung zur Auswahlsteuerung und Alternativgestaltung inkl. intensiver Tests für die Festlegung einzelner Komponenten unter gleichzeitiger Berücksichtigung vergleichbarer Implantate der aktuelle technische Stand der Implantattechnologie diskutiert werden.

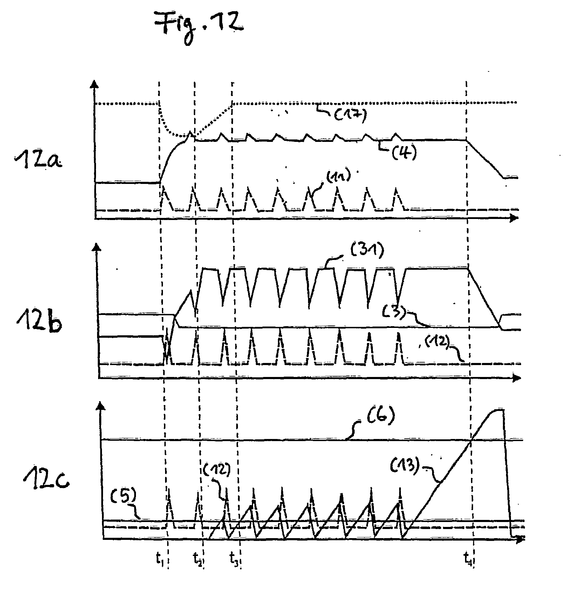

Die Ziffern in eckigen Klammern beziehen sich auf Fundstellen im Literaturverzeichnis am Ende dieser Beschreibung. Die Bezugszeichen beziehen sich auf die Figuren 12a bis 12c.

Ein aktuelles System eines künstlichen Blasenhalssphinkters, beispielhaft der AMS 800, bedient sich einer Handpumpe im Skrotum bzw. den großen Labien, mit der die den Harnfluss sperrende Manschette, die um die Harnröhre (Urethra) gelegt wird, aufgepumpt wird [6]. Problematisch bei diesem System ist die Einstellung des Urethraverschlussdruckes. Ein zu geringer Druck führt zu ungewolltem Urinabgang bei dynamischer Belastung, die durch Lachen, Husten, Niesen oder schweres Heben verursacht sein kann. Ein zu hoher Druck auf die Urethra über einen langen Zeitraum kann leicht zu schwarzem Gewebe, einer Nekrose, führen. Um diese Gefahr zu vermeiden, wird bislang in der Praxis eine leichte Belastungsinkontinenz mit all ihren negativen sozialen Folgen in Kauf genommen.

Das artifizielle feinsensorische Sphinkterimplantat, das erfindungsgemäß entwickelt wurde, ersetzt die Handpumpe durch eine aktive Hydraulik, die mit einer Feinsensorik und einer intelligenten Steuerung ausgestattet ist. Mit diesem System kann die aktive Drucktransmission unterstützt oder ersetzt werden. Die Sensorik überwacht primär die Differenz zwischen Blaseninnendruck und Cuffdruck. Bei dynamischer Belastung wird die Aktorik zur Erhöhung des Cuffdruckes benutzt, damit auch bei diesen erhöhten Druckvrhältnissen Kontinenz gewährleistet ist. Dadurch kann die normale Durchblutung des Urethragewebes kurzfristig beeinträchtigt sein. Das Implantat kann dabei zwischen einmaliger und andauernder dynamischer Belastung unterscheiden.

Steuerung

Die entscheidende Größe für die Funktion des artifiziellen feinsensorischen Sphinkterimplantats ist der Urethraverschlussdruck (31). Wird der Urethraverschlussdruck (31) negativ, tritt Urinabgang ein.

Das dem Urethraverschlussdruck (31) äquivalente Signal wird aus den beiden Absolutdrucksignalen des Cuffs (25) und der Harnblase ermittelt (Fig. 12a). Bei plötzlich auftretender dynamischer Belastung des Bauchinnenraumes bewegt sich dieses Signal schnell gegen Null. Um eine schnelle Reaktion des Implantats in dieser Situation sicherstellen zu können, werden folgende Maßnahmen getroffen:

Das Absinken des Urethraverschlussdruckes (31) wird nach unten durch eine schaltschwelle begrenzt, die oberhalb des Inkontinenzbereichs liegt und bei der Programmierung variiert werden kann.

Der Verlauf des Urethraverschlussdrucksignals (31) wird differenziert und der positive Anteil mit der unteren Schaltschwelle (12) addiert. Je schneller der Urethraverschlussdruck (31) absinkt, umso früher wird aufgrund dieser Maßnahme die Aktorik des Implantats aktiviert ($t_1$ (Fig. 12b).

Dieser zeitkritische Bereich wird mittels analoger elektronischer Komponenten realisiert, um eine maximale Reaktionsgeschwindigkeit zu ermöglichen. Die Aktivierung der Aktorik erfolgt durch einen Komparator, der das Signal des Urethraverschlussdruckes (31) mit der unteren Schaltschwelle (12) vergleicht.

Der Einsatz eines Vordruck-Behälters ermöglicht eine blitzartige Reaktion der Aktorik bei geringer Versorgungsspannung. Nach Beendigung des Druckausgleichs ($t_2$) wird der Vordruck (17) bis auf einen variierbaren Maximalwert $p_{max}$ erhöht (innerhalb $t_2$ bis $t_3$).

Die Absenkung des erhöhten Cuffdruckes unter die mit einer Hysterese ausgestatteten Normaldruckschwelle (3) erfolgt mikroprozessorgesteuert nach Beendigung der dynamischen Belastung. Um ein Maß für die dynamische Belastung zu erhalten wird die Differenz aus einem Offset-Wert (5), das bei der Programmierung ebenfalls variiert werden kann, und der unteren Schaltschwelle (12) integriert. Bei andauernder dynamischer Belastung wird somit das Integrationsergebnis (13) zickzackförmig nach unten gedrückt. Ohne dynamische Belastung steigt das Integrationsergebnis (13) bis zu einem Schwellenwert (6), bei dessen Erreichen das Absenken des Cuffdruckes ausgelöst wird ($t_4$) (Fig. 12c).

Die Normaldruckschwelle verhindert einen zu hohen Cuffdruck im Normalbetrieb. Diese Funktion wird, wie die Aktivierung der Aktorik, ebenfalls mit analogen elektronischen Komponenten realisiert.

Die analoge Schaltung beinhaltet 4 variierbare Parameter, die mittels D/A-Wandler beeinflusst werden, und zwar: die Geschwindigkeit der Differenzierung des Urethraverschlussdrucksignals, die additive Offset-Komponente des unteren Schwellenwertes sowie den Mittelwert und die Hysterese der Normaldruckschwelle. Die permanent aktivierte Elektronik beschränkt sich damit auf minimal 6 Operationsverstärker und einen Quad-Digital-Potentiometer der analogen Schaltung sowie auf die Signalkonditionierung und das RF-Empfangsmodul. Die gesamte Leistungsaufnahme der Elektronik beläuft sich mit dem Mikroprozessor im Power-Down-Modus auf unter 0,1 mW.

Programmier-Software

Die Programmierung des erfindungsgemäßen artifiziellen feinsensorischen Sphinkterimplantats erfolgt mittels einer externen Programmierstation. Über den bidirektionalen transkutanen Datentransfer werden der Urethraverschlussdruck sowie der Absolutdruck im Vordruckbehälter in die Programmier-Station übertragen. Die in-vivo-Druckmessung gepaart mit einer simultanen externen urodynamischen Untersuchung ermöglicht eine weitgehende Automatisierung der Implantat-Programmierung. Der Patient vollführt definierte körperliche Regungen, beispielsweise Aufstehen aus sitzender Position oder Husten. Aus den gesammelten Messdaten zusammen mit Erfahrungswerten aus klinischen Tests kann von der Software eine optimale Einstellung des Implantats evaluiert werden. Nachträglich erforderliche Anpassungen der Programmierung, beispielsweise an veränderte Lebensumstände des Patienten oder an verändertes Verhalten der implantierten Elektronik, sind auch manuell möglich.

Sensorik

Eine bestimmungsgemäße Funktion des artifiziellen Sphinkterimplantats erfordert den Einsatz von drei Drucksensoren, die an unterschiedlichen Orten und mit unterschiedlichen Voraussetzungen den Druck messen müssen:

> Im Vordruck-Behälter bietet sich der Einsatz eines Oberflächensensors an, der in die starre Grundplatte des Vordruck-Behälters integriert werden kann. Dieser kapazitive Drucksensor besteht ans einer Polysiliziummembran und einem Siliziumsubstrat [1]. Zwischen der Polysiliziummembran und dem Siliziumsubstrat wird bei der Herstellung ein Vakuum erzeugt, sodass ein Absolutdrucksensor entsteht. Zur Signalverstärkung lässt sich, bei dem geringen Durchmesser von 100-120 $\mu$m eines Sensorelementes, in Parallelschaltung ein Sensor-Array aufbauen [2].

Bei Einsatz eines Absolutdrucksensors im Vordruck-Behälter kann der Absolutdruck im Cuff auch mittels eines Differenzdrucksensors gemessen werden, der die Differenz zwischen Cuff- und Vordruck misst. Durch eine geeignete elektronische Schaltung kann ans dem Absolutdrucksignal des Vordruck-Behälters und dem Differenzdrucksignal zwischen Cuff und Vordruck-Behälter das Absolutdrucksignal des Cuffdruckes herausgefiltert werden. Dies ermöglicht den Einsatz eines kostengünstigen und zuverlässigen piezoresistiven Differenzdrucksensors, der über hydraulische Verbindungen sowohl mit dem Vordruck-Behälter als auch mit dem Cuff verbunden ist.

Zur Messung des Blaseninnendrucks wird ein Absolutdrucksensor, ähnlich einer Hirndrucksonde, in Blasennähe in das Gewebe eingebettet, um die Harnblase nicht punktieren oder öffnen zu müssen [5]. Der gemessene Druck entspricht möglicherweise nicht genau dem Blaseninnendruck, ist diesem aber äquivalent.

Elektronik

Die physikalischen Ausmaße sowie die Leistungsaufnahme der elektronischen Komponenten sind im artifiziellen Sphinkterimplantat nicht die kritischen Parameter, da beides im Vergleich zu den Eigenschaften der Aktorik vernachlässigbar ist. Dennoch wird eine Minimierung angestrebt, wenn auch mehr Wert auf Zuverlässigkeit und redundante Systeme gelegt werden kann.

Die für das artifizielle feinsensorische Sphinkterimplantat vorgesehene Steuerelektronik ähnelt bezüglich der Anforderungen der eines modernen Herzschrittmachers. Wie in einem Herzschrittmacher werden Sensorsignale von einem Mikroprozessor ausgewertet und in Aktionen des Implantats umgesetzt. Um den Stromverbrauch der Elektronik zu minimieren, wird der Mikroprozessor im Normalbetrieb im Power-Down-Modus betrieben. Der Komparator, der bei dynamischer Belastung die Aktorik aktiviert, aktiviert gleichzeitig den Mikroprozessor.

Der Einsatz eines über transkutane Datenübertragung programmierbaren Mikroprozessors hat den entscheidenden Vorteil, dass auf Veränderungen beispielsweise der Lebensgewohnheiten des Patienten oder im Verhalten der elektronischen Bauelemente flexibel reagiert werden kann. Der Einsatz analoger Halbleiterbauelemente, beispielsweise als Rechenschaltung zur Ermittlung des Cuff-Absolutdruckes, Wird dadurch weniger kritisch.

Mit einem Stromverbrauch von unter 1 $\mu$A pro Operationsverstärker können die analogen Komponenten problemlos permanent aktiviert bleiben. Der nur zeitweise aktive Mikroprozessor kann mit relativ hohen Taktfrequenzen betrieben werden, da die damit verbundene relativ hohe Leistungsaufnahme durch eine erheblich verbesserte Performance des Implantats leicht kompensiert werden kann.

Während bei einem Herzschrittmacher ein mit bis zu 1kV aufgeladener Kondensator geschaltet werden muss (meist mittels IGBTs), der sich über das menschliche Gewebe zwischen den Elektroden mit mehreren zehn Ampere innerhalb einiger Millisekunden entlädt, werden beim artifiziellen feinsensorischen Sphinkterimplantat bei einer Versorgungsspannung von 4,2 V maximale Dauerlastströme von etwa 100 mA pro Schalter geschaltet. Zu diesem Zweck bieten sich MOSFETs an, die direkt vom Mikroprozessor betrieben werden können. Damit die Zuverlässigkeit gewährleisten werden kann, werden pro Schalteinheit mehrere MOSFETs parallel geschaltet, um die Schaltaufgabe bei Ausfall einzelner Komponenten noch problemlos bewältigen zu können. Diese können in einem Multi-Chip-Modul (MCM) integriert werden.

Die Signalkonditionierung, die analoge Schaltung, die A/D-Wandlung, der interne Speicher und der Mikroprozessor mit einem multi-I/O-Modul können in einem Mixed-Signal ASIC, einem Application Specific Integrated Circuit, zusammengefasst werden [4]. Dies bietet eine extrem geringe Leistungsaufnahme, da anders als beim Einsatz eines handelsüblichen Mikroprozessors, keine ungenutzten Features brachliegen aber dennoch Strom verbrauchen. Diesem Vorteil steht ein hoher Entwicklungsaufwand gegenüber, der über Entwurf, Simulation und Fertigung zum gewünschten Ergebnis führt. Aus diesem Grund wird der Prototyp des Implantats mit einem handelsüblichen Microcontroller realisiert. Die Entwicklung eines ASIC wäre für einen Prototyp ein unverhältnismäßig hoher zeitlicher wie finanzieller Aufwand.

Energieträger ist in der Regel ein Lithium-Ionen-Akku, der über eine subkutan implantierte Induktionsspule aufgeladen wird. Der Lade-Controller des Lithium-Ionen-Akkupacks ist ein handelsüblicher IC, der beispielsweise in Mobilfunktelefonen zum Einsatz kommt. Er überwacht den Stromfluss zwischen dem Energie-Transfer-Modul und der Batterie, den er mit einem MOSFET beeinflussen kann. Beendigung, Abbruch und Fehler werden dem Mikroprozessor gemeldet.

Aktorik

Die Miniaturisierung der implantierbaren Aktorik erfordert Komponenten, die den hohen Ansprüchen heutiger Medizintechnik wie Biokompatibilität, Langlebigkeit und Zuverlässigkeit gerecht werden. In der Implantationsmedizin gehören Silikone und Polyurethane, die durch ihre Körperverträglichkeit gekennzeichnet sind, zu den am häufigsten genutzten Materialien. In mechanischen wie thermischen Dauerbelastungstests stellte sich heraus, dass Silikone und Polyurethane durchaus gleichwertige Eigenschaften aufweisen. Mit mehr als 5 Millionen Belastungszyklen wurde die Haltbarkeit für den Einsatz im Menschen simuliert und betätigt.

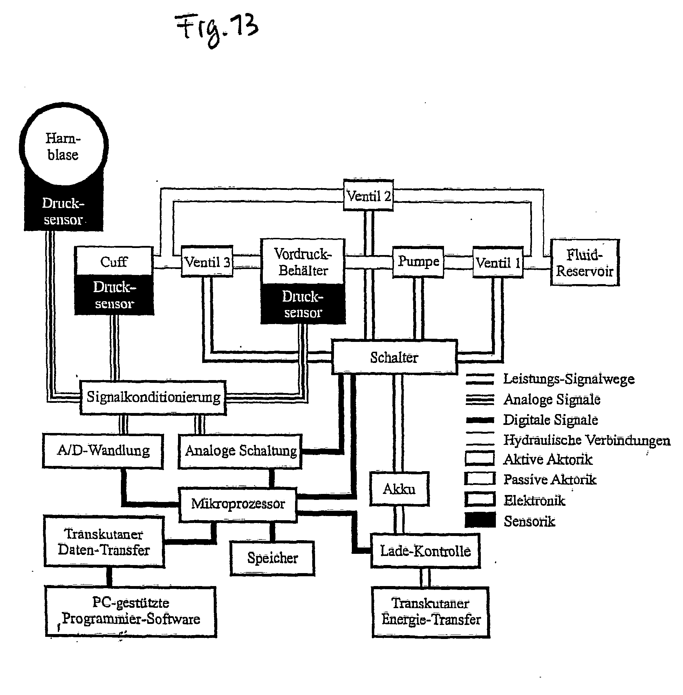

Die Aktorik des artifiziellen feinsensorischen Sphinkterimplantäts besteht aus elektrisch aktiven Komponenten, den Ventilen und der Pumpe, und aus passiven, einer Vordruckkammer, einer aufpumpbaren Manschette und den hydraulischen Verbindungen (Figur 13).

Diese Komponenten müssen für den Einsatz im artifiziellen feinsensorischen Sphinkterimplantat speziell angepasst werden. Industrielle Pumpen und Ventile sind größtenteils für Drücke von 10bar und mehr ausgelegt. Da in dem Implantat maximale Drücke von unter 500 mbar auftreten, ist eine angepasste Dimensionierung dieser Komponenten sinnvoll.

Der Vordruck-Behälter besteht aus einer starren Grundplatte, über die eine elastische Membran gespannt wird. Durch den flachen Aufbau kann der Vordruck-Behälter so in die Außenhülle des Implantats integriert werden, dass sich die elastische Membran bei Druckerhöhung nach außen wölben kann.

Die entscheidende Funktion des artifiziellen feinsensorischen Sphinkterimplantats ist die blitzartige Erhöhung des Cuffdruckes bei plötzlichem Absinken des Urethraverschlussdrukkes. Um die Zeit zwischen Aktivierung der Aktorik und Erreichen eines sicheren Cuffdruckes so gering wie möglich zu halten, wird ein erhöhter Vordruck bereitgestellt, der über das Ventil 3 auf das Cuff übertragen werden kann. Da die Elektronik Reaktionszeiten im Mikro- bis Nanosekundenbereich erreicht, ist der entscheidende Gewinn an Reaktionszeit bei der Optimierung des Systems aus Vordruck-Behälter, Ventil 3 und der hydraulischen Verbindung zu erwarten. Wenn beispielsweise zur Erhöhung des Cuffdruckes von 70 mbar auf 100 mbar ein Fluss von 0,2 ml benötigt wird und die Druckdifferenz zwischen Cuff und Vordruck- Behälter vor dem Druckausgleich $\Delta p$ ist, ergibt sich mit einem Öffnungsradius des Schlauches r folgende Proportionalität (G1. 1):

$$ t \sim \frac{1}{r^2 \cdot \Delta p} $$

Reaktionszeiten von Aktivierung der Aktorik bis zur Beendigung des Druckausgleichs sind unter 10 ms realisierbar. Hierzu ist eine Druckdifferenz von $\Delta p = 400$ mbar und ein Innenradius der hydraulischen Verbindungen von etwa r = 1,0 mm nötig. Mit einer weiteren Vergrößerung des Innenradius' r können noch kürzere Reaktionszeiten realisiert werden.

Desweiteren verursacht die Massenträgheit des Ventils 3 eine weitere Verzögerung, die mit zunehmendem Öffnungsradius r steigt.

Die Absenkung des Cuffdruckes erfolgt über Ventil 2 in das Reservoir. Bei der Dimensionierung des Ventils 2 und der hydraulischen Verbindungen zwischen Cuff und Reservoir muss darauf geachtet werden, dass das Absenken des Cuffdruckes nicht zu schnell erfolgt. Ist dies der Fall, kann aufgrund der Trägheit des Ventils und der entgegenkommenden unteren Schaltschwelle sofort wieder die Erhöhung des Cuffdruckes ausgelöst werden. Mit einer entsprechenden Wahl der Querschnittsöffnung der hydraulischen Verbindungen kann diese Gefahr aber vermieden werden.

Energie- und Datenübertragung

Bezüglich der Energie- und Datenübertragung lassen sich die meisten Gemeinsamkeiten mit der künstlichen Harnblase [6] [7] [8] feststellen. Anders als beispielsweise beim künstlichen Herz dient die Energieübertragung beim artifiziellen feinsensorischen Sphinkterimplantat nur dem Laden des implantierten Akkus und die Datenübertragung wird hauptsächlich zur Programmierung des Mikroprozessors verwendet. Des weiteren wird zur Miktionskontrolle ein weiterer Signalweg verwendet, der für den Patienten unkompliziert zu bedienen ist. Aus diesen Rahmenbedingungen wurde für den Einsatz im artifiziellen feinsensorischen Sphinkterimplantat folgende Konfiguration der Energie- und Datenübertragung entworfen:

Das Laden des implantierten Akkus erfolgt induktiv durch Auflegen eines Ladegeräts auf eine subkutan implantierte Induktionsspule [3]. Um eine schnelle bidirektionale Datenübertragung zu ermöglichen, wird in der Mitte der Induktionsspule ein IR-Sende/Empfangs-Modul platziert. Diese optische Datenübertragung erfordert, wie die induktive Energieübertragung, das Auflegen des externen Gegenstücks direkt auf die Haut.

Diese Funktion kann damit in das externe Ladegerät integriert werden.
Die Miktionskontrolle erfolgt über eine unidirektionale RF-Datenübertragung. Dies ermöglicht dem Patienten die komfortable und unkomplizierte Bedienung des Implantats. Übermittelt werden der Miktionswunsch sowie ein Signal zur Beendigung der Miktion.

Ergebnisse

Durch eine geeignete Konstruktion der Aktorik sowie eine entsprechende Auslegung der Elektronik wurde es erreicht, eine blitzartige Reaktion der Hydraulik mit der geringen Versorgungsspannung von 4,2 V und das komplexe Verhalten der Elektronik mit der geringen Leistungsaufnahme von unter 0,1 mW im Power-Down-Modus des Mikroprozessors zu erreichen. Das Verhalten des Implantats ist dabei derart variabel gestaltet, dass es einem enorm breiten Spektrum an Anforderungen gerecht werden kann.

Literaturverzeichnis

[1] M. Kandler, J. Eichholz, Y. Manoli, W. Mokwa, "CMOS compatible capacitive pressure sensor with readout electronics", International Conference on Micro Electro, Opto, Mechanic Systems and Components, Micro System Technologies, pp. 574-580, 1990

[2] H. Dudaicevs, Y. Manoli, W. Mokwa, M. Schmidt, E. Spiegel, "A fully integrated surface micromachined pressure sensor with low temperature dependence", Transducers, Digest of technical papers, pp. 616-619, 1995

[3] H. Wassermann, ,"Drahtlose Energie- und Signalübertragung mit gleichzeitiger Disloziervorkehrung und -vorrichtung bei Unzugänglichkeit einer Seite und bei undurchsichtigem Dielektrikum", Technisches FB-Kompendium, 1992

[4] R. Lerch, E. Spiegel, R. Kakerow, R. Hakenes, H. Kappert, H. Kohlhaas, N. Kordas, M. Buchmann, T. Franke, Y. Manoli, J. Müller, "A programmable mixed-signal ASIC for data-acquisition systems in medical implants", International Solid-State Circuits Conference, Digest of technical papers, pp. 160-161, 1995

[5] A. Atala, M.R. Freeman, J.P. Vacanti, J. Shepard, A.B. Retik, "Implantation in vivo and retrieval of artificial structures consisting of rabbit and human urothelium and human bladder muscle", J.Urol. 150, pp. 608-612, 1993

[6] D. Jocham und K. Miller "Praxis der Üralogie II", Stuttgart: Georg Thieme, 1994/2002

[7] H. Wassermann, "Künstliches harnableitendes System", Medizintechnik in Bayern, vol. 2, pp. 57-61, 2002

[8] R. Stölting, "künstliche Harnblase - Klinische Tests stehen noch aus", medizin report, vol 2, pp. 22-23

[9] H. Wassermann, "Artificial Urinary Diversion System", Bavarian Medical Technologies, vol. 2, pp. 53-57, 2002

**Patentansprüche**

1. System zur elektronischen Steuerung eines artifiziellen feinsensorischen Sphinkterimplantats, **dadurch gekennzeichnet, dass** in dem System mindestens ein sich analog der Differenz zwischen Blaseninnendruck (11) und Cuffdruck (4) verhaltendes Sensorsignal bzw. Sensorwert (1) in einer analogen oder digitalen elektronischen Schaltung mittels Rechen- und Komparatorelementen so umgewandelt und mit Referenzwerten verglichen wird, dass eine Aktorik derart gesteuert wird, dass sich der Cuffdruck bzw. der Differenzdruck zwischen Cuff und Harnblase entweder in einem durch zwei Schwellenwerte begrenzten niedrigen Bereich bewegt oder oberhalb eines bestimmten Sicherheitsdruckes bzw. bei Miktion unterhalb davon.

2. System nach Anspruch 1, **dadurch gekennzeichnet, dass** das Rechenelement der elektronischen Schaltung umfasst:

(a) ein Differentiatorelement, dessen Ausgangssignal bzw. Ausgangswert (14) der Differentiation des Verlaufes des Sensorsignals bzw. des Sensorwertes (1) entspricht,

(b) ein Addiererelement mit einem monostabilen Offsetsignal bzw. Offsetwert, dessen Ausgangssignal bzw. Ausgangswert (12) dem um das monostabile Offsetsignal bzw. den monostabilen Offsetwert erhöhten Ausgangssignal bzw. Ausgangswert (14) des Differentiatorelementes entspricht,

(c) ein Integratorelement mit einem monostabilen Referenzsignal bzw. einem monostabilen Referenzwert (5) und einem konstanten Startwert, bei dem wahlweise und

(i) das monostabile Referenzsignal bzw. der monostabile Referenzwert (5) ungleich dem monostabilen Offsetsignal bzw. Offsetwert des Addiererelementes ist und derart gewählt wird, dass das Ausgangssignal bzw. der Ausgangswert (12) des Addiererelementes bei Aktivität das monostabile Referenzsignal bzw. den monostabilen Referenzwert (5) kreuzt, wobei das Integratorelement die Differenz zwischen dem Ausgangssignal bzw. dem Ausgangswert (12) des Addiererelementes und dem monostabilen Referenzsignal bzw. dem monostabilen Referenzwert (5) derart kontinuierlich oder numerisch integriert, dass sich bei geringer Aktivität des Ausgangssignals bzw. des Ausgangswertes (14) des Differentiatorelementes das Ausgangssignal bzw. der Ausgangswert (13) des Integratorelementes vom Startwert entfernt und bei hoher Aktivität des Ausgangssignals bzw. des Ausgangswertes (14) des Differentiatorelementes auf den Startwert zubewegt, oder

(ii) das monostabile Referenzsignal bzw. der monostabile Referenzwert (5) ungleich dem Ausgangssignal bzw. Ausgangswert (14) des Differentiatorelementes bei geringer Aktivität ist und derart gewählt wird, dass das Ausgangssignal bzw. der Ausgangswert (14) des Differentiatorelementes bei Aktivität das monostabile Referenzsignal bzw. den monostabilen Referenzwert (5) kreuzt, wobei das Integratorelement die Differenz zwischen dem Ausgangssignal bzw. dem Ausgangswert (14) des Differentiatorelementes und dem monostabilen Referenzsignal bzw. dem monostabilen Referenzwert (5) derart kontinuierlich oder numerisch integriert, dass sich bei geringer Aktivität des Ausgangssignals bzw. des Ausgangswertes (14) des Differentiatorelementes das Ausgangssignal bzw. der Ausgangswert (13) des Integratorelementes kontinuierlich vom Startwert entfernt und bei hoher Aktivität des Ausgangssignals bzw. des Ausgangswertes (14) des Differentiatorelementes auf den Startwert zubewegt,

(d) ein Komparatorelement mit einem bistabilen Schwellensignal bzw. Schwellenwert (3), wodurch für den Fall der Parität bzw. der Kreuzung des Sensorsignals bzw. Sensorwertes (1) mit dem bistabilen Schwellensignal bzw. dem bistabilen Schwellenwert (3) dieses Schwellensignal bzw. dieser Schwellenwert (3) um den durch die Hysterese bedingten Betrag abgesenkt bzw. angehoben und **dadurch** ein Signal ausgelöst wird, das die Aktorik solange zur Absenkung (16) des Cuffdruckes (4) veranlasst bis durch nochmalige Parität bzw. nochmaliges Kreuzen des Sensorsignals (1) mit dem bistabilen Schwellensignal bzw. dem bistabilen Schwellenwert (3) ein Signal ausgelöst wird, das die Aktorik zur Beendigung der Absenkung (16) des Cuffdruckes (4) veranlasst und das bistabile Schwellensignal bzw. den bistabilen Schwellenwert (3) um den durch die Hysterese bedingten Betrag anhebt bzw. absenkt, und

(e) ein Komparatorelement mit einem monostabilen Schwellensignal bzw. Schwellenwert (2), wodurch für den Fall der Parität bzw. der Kreuzung des Sensorsignals bzw. Sensorwertes (1) mit dem monostabilen Schwellensignal bzw. dem monostabilen Schwellenwert (2) ein Signal ausgelöst wird, das die Aktorik zur Beendigung der Erhöhung (15) des Cuffdruckes (4) veranlasst und das Integratorelement derart aktiviert, dass bei Aktivierung das Ausgangssignal bzw. der Ausgangswert (13) des Integratorelementes dem konstanten Startwert entspricht, insbesondere **dadurch gekennzeichnet, dass** das Sensorsignal bzw. der Sensorwert (1) mit dem Ausgangssignal bzw. dem Ausgangswert (12) des Addiererelementes derart verglichen wird, dass für den Fall der Parität bzw. der Kreuzung des Sensorsignals bzw. des Sensorwertes (1) mit dem Ausgangssignal bzw. dem Ausgangswert (12) des Addiererelementes ein Signal ausgelöst wird, das die Aktorik zur Erhöhung (15) des Cuffdruckes (4) veranlasst und das Komparatorelement nach Anspruch 26 (d) außer Kraft setzt, insbesondere **dadurch gekennzeichnet, dass** für den Fall der Parität bzw. der Kreuzung des Ausgangssignals bzw. des Ausgangswertes (13) des Integratorelementes mit dem monostabilen Schwellensignal bzw. Schwellenwert (6) ein Signal ausgelöst wird, das die Aktorik zur Senkung (16) des Cuffdruckes (4) veranlasst und/oder das Komparatorelement nach Anspruch 26 (d) aktiviert, insbesondere **dadurch gekennzeichnet, dass** ferner ein Verzögerungselement vorgesehen ist, das bei Auslösen eines Druckausgleichs zwischen einem Vordruck-Behälter (29) und einem Cuff (25) durch ein Komparatorelement aktiviert wird und bei Erreichen der eingestellten Verzögerungszeit ein Signal auslöst, das die Aktorik zur Beendigung des Druckausgleichs zwischen dem Vordruck-Behälter (29) und dem Cuff (25) veranlasst und das Integratorelement derart aktiviert, dass bei Aktivierung das Ausgangssignal bzw. der Ausgangswert (13) des Integratorelementes dem konstanten Startwert entspricht, insbesondere **dadurch gekennzeichnet, dass** das monostabile Offsetsignal bzw. der monostabile Offsetwert durch Parameter-Variation von außen variiert werden kann, insbesondere **dadurch gekennzeichnet, dass** das bistabile Schwellensignal bzw. der bistabile Schwellenwert (3) durch Parameter-Variation von außen variiert werden kann, insbesondere **dadurch gekennzeichnet, dass** das monostabile Referenzsignal bzw. der monostabile Referenzwert (5) durch Parameter-Variation von außen variiert werden kann, insbesondere **dadurch gekennzeichnet, dass** das monostabile Schwellensignal bzw. der monostabile Schwellenwert (2) durch Parameter-Variation von außen variiert werden kann, insbesondere **dadurch gekennzeichnet, dass** die Parameter-Variation durch Programmierung der Elektronik über Infrarot-Übertragung von außen bewirkt wird.

3. System zur Kontrolle der Miktion der elektronischen Schaltung nach Anspruch 1 oder 2, **dadurch gekennzeichnet, dass** zur Aktivierung der Miktion ein externes Signal die Aktorik zur Senkung des Urethra-Verschlussdruckes veranlasst und die elektronische Schaltung bis auf das Komparatorelement nach Anspruch 26 (d) deaktiviert und zur Deaktivierung der Miktion ein externes Signal die Aktorik zur Erhöhung (15) des Cuffdruckes (4) veranlasst bis für den Fall der Parität bzw. der Kreuzung des Sensorsignals (1) mit dem bistabilen Schwellensignal bzw. Schwellenwert (3) ein Signal ausgelöst wird, das die elektronische Schaltung aktiviert.

4. System nach Anspruch 3, **dadurch gekennzeichnet, dass** das externe Signal zur Aktivierung oder Deaktivierung der Miktion durch Infrarot-Übertragung oder Funk oder Induktion übermittelt wird, insbesondere **dadurch gekennzeichnet, dass** die Energieversorgung des artifiziellen feinsenorischen Sphinkterimplantats von außen durch Induktion erfolgt.

**Claims**

1. A system for electronically controlling an artificial fine-sensory sphincter implant, **characterised in that** in the system at least one sensor signal or sensor value (1) acting analogously to the difference between the internal pressure of the bladder (11) and the cuff pressure (4) is converted in an analogue or digital electronic circuit by means of calculation and comparator elements and compared to reference values **in that** an actuator is controlled such that the cuff pressure or the pressure difference between the cuff and the urinary bladder is either moved in a low region defined by two threshold values or above a specific safety pressure or upon micturition below the latter.

2. The system according to Claim 1, **characterised in that** the calculation element of the electronic circuit comprises:

   (a) a differentiator element the output signal or output value (14) of which corresponds to the differentiation of the development of the sensor signal or of the sensor value (1),
   (b) an adder element with a monostable offset signal or offset value the output signal or output value (12) of which corresponds to the output signal or output value (14) of the differentiator element increased by the monostable offset signal or the monostable offset value,
   (c) an integrator element with a monostable reference signal or a monostable reference value (5) and a constant start value with which optionally
   and
   (i) the monostable reference signal or the monostable reference value (5) is not equal to the monostable offset signal or offset value of the adder element and is chosen such that the output signal or the output value (12) of the adder element crosses the monostable reference signal or the monostable reference value (5) when there is activity, the integrator element continuously or numerically integrating the difference between the output signal or the output value (12) of the adder element and the monostable reference signal or the monostable reference value (5) such that with a small amount of activity of the output signal or of the output value (14) of the differentiator element the output signal or the output value (13) of the integrator element is removed from the start value and with a large amount of activity of the output signal or of the output value (14) of the differentiator element the output signal or the output value (13) of the integrator element moves towards the start value,

or

(ii) the monostable reference signal or the monostable reference value (5) is not equal to the output signal or output value (14) of the differentiator element with a small amount of activity and is chosen such that the output signal or the output value (14) of the differentiator element crosses the monostable reference signal or the monostable reference value (5) when there is activity, the integrator element continuously or numerically integrating the difference between the output signal or the output value (14) of the differentiator element and the monostable reference signal or the monostable reference value (5) such that with a small amount of activity of the output signal or of the output value (14) of the differentiator element the output signal or the output value (13) of the integrator element is continuously removed from the start value and with a large amount of activity of the output signal or of the output value (14) of the differentiator element the output signal or the output value (13) of the integrator element moves towards the start value,

(d) a comparator element with a bistable threshold signal or threshold value (3), by means of which, in the event of parity or crossover of the sensor signal or sensor value (1) with the bistable threshold signal or the bistable threshold value (3) this threshold signal or this threshold value (3) is decreased or increased by the amount conditional upon the hysteresis, and in this way a signal is triggered which causes the actuator to decrease (16) the cuff pressure (4) until, due to parity or crossover of the sensor signal (1) once again with the bistable threshold signal or the bistable threshold value (3) a signal is triggered which causes the actuator to end the decrease (16) of the cuff pressure (4), and increases and decreases the bistable threshold signal or the bistable threshold value (3) by the amount conditional upon the hysteresis, respectively,

and

(e) a comparator element with a monostable threshold signal or threshold value (2) by means of which, in the event of parity or crossover of the sensor signal or sensor value (1) with the monostable threshold signal or the monostable threshold value (2) a signal is triggered which causes the actuator to stop increasing (15) the cuff pressure (4) and the integrator element is activated such that upon activation the output signal or the output value (13) of the integrator element corresponds to the constant start value, in particular **characterised in that** the sensor signal or the sensor value (1) is compared to the output signal or the output value (12) of the adder element such that in the event of parity or crossover of the sensor signal or of the sensor value (1) with the output signal or the output value (12) of the adder element a signal is triggered which causes the actuator to increase (15) the cuff pressure (4) and overrides the comparator element according to Claim 26 (d), in particular **characterised in that** in the event of parity or crossover of the output signal or of the output value (13) of the integrator element with the monostable threshold signal or threshold value (6) a signal is triggered which causes the actuator to reduce (16) the cuff pressure (4) and/or activates the comparator element according to Claim 26 (d), in particular **characterised in that** furthermore a delay element is provided which upon triggering of a pressure equalisation between a primary pressure container (29) and a cuff (25) is activated by a comparator element and upon reaching the set delay time triggers a signal which causes the actuator to end the pressure equalisation between the primary pressure container (29) and the cuff (25) and activates the integrator element such that upon activation the output signal or the output value (13) of the integrator element corresponds to the constant start value, in particular **characterised in that** the monostable offset signal or the monostable offset value can be varied from the outside by parameter variation, in particular **characterised in that** the bistable threshold signal or the bistable threshold value (3) can be varied from the outside by parameter variation, in particular **characterised in that** the monostable reference signal or the monostable reference value (5) can be varied from the outside by parameter variation, in particular **characterised in that** the monostable threshold signal or the monostable threshold value (2) can be varied from the outside by parameter variation, in particular **characterised in that** the parameter variation can be brought about from the outside by programming the electronics by infra red transmission.

3. The system for controlling the micturation of the electronic circuit according to Claim 1 or 2, **characterised in that** in order to activate micturation an external signal causes the actuator to reduce the urethra closing pressure and deactivates the electronic circuit except for the comparator element according to Claim 26 (d) and in order to deactivate micturation an external signal causes the actuator to increase (15) the cuff pressure (4) until, in the event of parity or crossover of the sensor signal (1) with the bistable threshold signal or threshold value (3), a signal is triggered which activates the electronic circuit.

**4.** The system according to Claim 3, **characterised in that** the external signal for activation or deactivation of micturation is transmitted by infrared transmission or radio or induction, in particular **characterised in that** the energy supply of the artificial fine-sensory sphincter implant takes place from the outside by induction.

**Revendications**

**1.** Système de commande électronique d'un implant sphinctérien artificiel à fine détection, **caractérisé en ce que,** dans le système, au moins un signal de détection ou une valeur de détection se comportant de manière analogue à la différence existant entre la pression régnant à l'intérieur de la vessie (11) et la pression du brassard compressif (4) est transformé, dans un circuit électronique analogique ou numérique, au moyen d'éléments de calcul et de comparaison et est comparé à des valeurs de référence de telle façon qu'un acteur soit commandé de manière que la pression du brassard compressif ou la pression différentielle entre le brassard compressif et la vessie se déplace soit dans une plage basse délimitée par deux valeurs seuils, soit au-dessus d'une pression de sécurité déterminée ou, lors de la miction, au-dessous de celle-ci.

**2.** Système selon la revendication 1, **caractérisé en ce que** l'élément de calcul du circuit électronique comprend:

(a) un élément différentiateur dont le signal de sortie ou la valeur de sortie (14) correspond à la différentiation du cheminement du signal de détection ou de la valeur de détection (1),
(b) un élément additionneur ayant un signal de décalage monostable ou une valeur de décalage monostable dont le signal de sortie ou la valeur de sortie (12) correspond au signal de sortie ou à la valeur de sortie (14) de l'élément différentiateur additionné du signal de décalage monostable ou de la valeur de décalage monostable,
(c) un élément intégrateur ayant un signal de référence monostable ou une valeur de référence monostable (5) et une valeur de départ constante, dans lequel soit
et
(i) le signal de référence monostable ou la valeur de référence monostable (5) diffère du signal de décalage monostable ou de la valeur de décalage monostable de l'élément additionneur et est choisi de telle sorte que le signal de sortie ou la valeur de sortie (12) de l'élément additionneur croise, lorsqu'il y a activité, le signal de référence monostable ou la valeur de référence monostable (5), l'élément intégrateur intégrant la différence existant entre le signal de sortie ou la valeur de sortie (12) de l'élément additionneur et le signal de référence monostable ou la valeur de référence monostable (5) de manière continue ou numérique de telle sorte que le signal de sortie ou la valeur de sortie (13) de l'élément intégrateur s'éloigne de la valeur de départ lorsqu'il y a réduction de l'activité du signal de sortie ou de la valeur de sortie (14) de l'élément différentiateur et, en cas d'activité accrue du signal de sortie ou de la valeur de sortie (14) de l'élément différentiateur, qu'il s'approche de la valeur de départ,
ou
(ii) le signal de référence monostable ou la valeur de référence monostable (5) diffère du signal de sortie ou de la valeur de sortie (14) de l'élément différentiateur en cas de réduction d'activité et est choisi de telle sorte que le signal de sortie ou la valeur de sortie (14) de l'élément différentiateur croise, lorsqu'il y a activité, le signal de référence monostable ou la valeur de référence monostable (5), l'élément intégrateur intégrant la différence existant entre le signal de sortie ou la valeur de sortie (14) de l'élément différentiateur et le signal de référence monostable ou la valeur de référence monostable (5) de manière continue ou numérique de telle sorte que, lorsqu'il y a réduction d'activité du signal de sortie ou de la valeur de sortie (14) de l'élément différentiateur, le signal de sortie ou la valeur de sortie (13) de l'élément intégrateur s'éloigne en continu de la valeur de départ et, en cas d'activité accrue du signal de sortie ou de la valeur de sortie (14) de l'élément différentiateur, il s'approche de la valeur de départ,
(d) un élément comparateur ayant un signal seuil bistable ou une valeur seuil bistable (3), moyennant lequel, dans le cas d'une parité ou d'un croisement du signal de détection ou de la valeur de détection (1) avec le signal seuil bistable ou la valeur seuil bistable (3), ce signal seuil ou cette valeur seuil (3) subit un abaissement ou une augmentation correspondant à la quantité due à l'hystérésis, ce qui entraîne la production d'un signal qui amène l'acteur à baisser (16) la pression du brassard compressif (4) jusqu'à ce que le retour de la parité ou du croisement du signal de détection (1) avec le signal seuil bistable ou la valeur seuil bistable (3) entraîne la production d'un signal qui amène l'acteur à terminer l'abaissement (16) de la pression du brassard compressif (4) et qui entraîne une élévation ou un abaissement du signal seuil bistable ou de la valeur seuil bistable (3) correspondant à la quantité due à l'hystérésis, et

(e) un élément comparateur ayant un signal seuil monostable ou une valeur seuil monostable (2), moyennant lequel, dans le cas d'une parité ou d'un croisement du signal de détection ou de la valeur de détection (1) avec le signal seuil monostable ou la valeur seuil monostable (2), il est produit un signal qui amène l'acteur à terminer l'augmentation (15) de la pression du brassard compressif (4) et active l'élément intégrateur de telle sorte que lors d'une activation, le signal de sortie ou la valeur de sortie (13) de l'élément intégrateur corresponde à la valeur de départ constante, notamment **caractérisé en ce que** le signal de détection ou la valeur de détection (1) est comparé(e) au signal de sortie ou à la valeur de sortie (12) de l'élément additionneur de sorte que, dans le cas d'une parité ou d'un croisement du signal de détection ou de la valeur de détection (1) avec le signal de sortie ou la valeur de sortie (12) de l'élément additionneur, il est produit un signal qui amène l'acteur à augmenter (15) la pression du brassard compressif (4) et qui désactive l'élément comparateur selon la revendication 26 (d), notamment **caractérisé en ce que**, dans le cas d'une parité ou d'un croisement du signal de sortie ou de la valeur de sortie (13) de l'élément intégrateur avec le signal seuil monostable ou la valeur seuil monostable (6), il est produit un signal qui amène l'acteur à abaisser (16) la pression du brassard compressif (4) et/ou qui active l'élément comparateur selon la revendication 26 (d), notamment **caractérisé en ce qu'**il est encore prévu un élément retardateur qui est activé par un élément comparateur lors du déclenchement d'une compensation de pression entre un réservoir à précompression (29) et un brassard compressif (25) et qui, une fois que le temps de retard défini est atteint, produit un signal qui amène l'acteur à terminer la compensation de pression entre le réservoir à précompression (29) et le brassard compressif (25) et qui active l'élément intégrateur de telle sorte que, lors de l'activation, le signal de sortie ou la valeur de sortie (13) de l'élément intégrateur corresponde à la valeur de départ constante, notamment **caractérisé en ce que** le signal de décalage monostable ou la valeur de décalage monostable peut être modifié(e) depuis l'extérieur par une variation de paramètres, notamment **caractérisé en ce que** le signal seuil bistable ou la valeur seuil bistable (3) peut être modifié(e) depuis l'extérieur par une variation de paramètres, notamment **caractérisé en ce que** le signal de référence monostable ou la valeur de référence monostable (5) peut être modifié(e) depuis l'extérieur par une variation de paramètres, notamment **caractérisé en ce que** le signal seuil monostable ou la valeur seuil monostable (2) peut être modifié(e) depuis l'extérieur par une variation de paramètres, notamment **caractérisé en ce que** la variation de paramètres peut être provoquée depuis l'extérieur par programmation du dispositif électronique par transmission infrarouge.

3. Système pour le contrôle de la miction du circuit électronique selon la revendication 1 ou 2, **caractérisé en ce que,** pour activer la miction, un signal externe amène l'acteur à abaisser la pression de fermeture de l'urètre et désactive le circuit électronique à l'exception de l'élément comparateur selon la revendication 26 (d) et, pour désactiver la miction, un signal externe amène l'acteur à élever (15) la pression du brassard compressif (4) jusqu'à ce que, pour le cas de la parité ou du croisement du signal de détection (1) avec le signal seuil bistable ou la valeur seuil bistable (3), il soit produit un signal qui active le circuit électronique.

4. Système selon la revendication 3, **caractérisé en ce que** le signal externe d'activation ou de désactivation de la miction est transmis par transmission infrarouge, par radio ou par induction, notamment **caractérisé en ce que** l'implant sphinctérien artificiel à fine détection est alimenté en énergie depuis l'extérieur par induction.

Fig. 1A

Harnblase
Steuerungseinheit
R2 R1
V1 V2 V3
S1 S2 S3
Pumpe
Z1 Z2
1 3 5 7 11

EP 1 513 466 B1

Fig. 1 B

Fig. 2

**Fig. 3**

Fig. 4

Differenzdrucksignal

1

32

Druckausgleichsschwelle

Komparatorglied

36

Differentiatorfunktion

10

Integratorfunktion
37

14

Vordrucksignal

33

7 8

Monostabiles
Offsetsignal

Addiererfunktion

Vordruck=
schwelle

5

12

36

Monostabiles
Referenzsignal

Komparatorglied

Komparatorglied

36

34

3

13

Integratorfunktion

6

Normaldruck=
schwelle

Integrations=
schwelle

Komparatorglied

36

Komparatorglied

36

35

Aktorik

Parameter-Variation

Fig. 5a

Fig. 5b

Fig. 5c

Fig. 5d

Fig. 5e

Fig. 6a

Fig. 6b

Fig. 6c

Fig. 6d

Fig. 6e

Fig. 6f

Fig. 6g

26

Fig. 7a

Fig. 7b

Fig. 7c

Fig. 7d

Fig. 7e

Fig. 8a

Fig. 8b

Fig. 8c

Fig. 8d

Fig. 8e

Fig. 8f

Fig. 8g

Fig. 9a

Fig. 9b

Fig. 10

25
Cuff

23

24

26
Ventil

24

27
Pumpe

24

28
Fluid-Reservoir

Verfahren zur Regulation des Urethra-
Verschlussdruckes

Fig. 11

Verfahren zur Regulation des Urethra-Verschlussdruckes
mit Vordruckbehälter

Fig. 12

12a

12b

12c

$t_1$    $t_2$    $t_3$               $t_4$

Fig. 13

Harnblase

Drucksensor

Ventil 2

Cuff

Drucksensor

Ventil 3

Vordruck-
Behälter

Drucksensor

Pumpe

Ventil 1

Fluid-
Reservoir

Schalter

Signalkonditionierung

A/D-Wandlung

Analoge Schaltung

Akku

Mikroprozessor

Transkutaner
Daten-Transfer

Speicher

Lade-Kontrolle

PC-gestützte
Programmier-Software

Transkutaner
Energie-Transfer

Leistungs-Signalwege
Analoge Signale
Digitale Signale
Hydraulische Verbindungen
Aktive Aktorik
Passive Aktorik
Elektronik
Sensorik

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4331658

- WO 0150833 A

**In der Beschreibung aufgeführte Nicht-Patentliteratur**

- **M. KANDLER ; J. EICHHOLZ ; Y. MANOLI ; W. MOKWA.** CMOS compatible capacitive pressure sensor with readout electronics. *International Conference on Micro Electro, Opto, Mechanic Systems and Components, Micro System Technologies,* 1990, 574-580
- **H. DUDAICEVS ; Y. MANOL ; W. MOKWA ; M. SCHMIDT ; E. SPIEGEL.** A fully integrated surface micromachined pressure sensor with low temperature dependence. *Transducers, Digest of technical papers,* 1995, 616-619
- **H. WASSERMANN.** Drahtlose Energie- und Signalübertragung mit gleichzeitiger Disloziervorkehrung und -vorrichtung bei Unzugänglichkeit einer Seite und bei undurchsichtigem Dielektrikum. *Technisches FB-Kompendium,* 1992
- **R. LERCH ; E. SPIEGEL ; R. KAKEROW ; R. HAKENES ; H. KAPPERT ; H. KOHLHAAS ; N. KORDAS ; M. BUCHMANN ; T. FRANKE ; Y. MANOLI.** A programmable mixed-signal ASIC for data-acquisition systems in medical implants. *International Solid-State Circuits Conference, Digest of technical papers,* 1995, 160-161

- **A. ATALA ; M.R. FREEMAN ; J.P. VACANTI ; J. SHEPARD ; A.B. RETIK.** Implantation in vivo and retrieval of artificial structures consisting of rabbit and human urothelium and human bladder muscle. *J.Urol.,* 1993, vol. 150, 608-612
- **D. JOCHAM ; K. MILLER.** Praxis der Üralogie II. Georg Thieme, 1994
- **H. WASSERMANN.** Künstliches harnableitendes System. *Medizintechnik in Bayern,* 2002, vol. 2, 57-61
- **R. STÖLTING.** künstliche Harnblase - Klinische Tests stehen noch aus. *medizin report,* vol. 2, 22-23
- **H. WASSERMANN.** Artificial Urinary Diversion System. *Bavarian Medical Technologies,* 2002, vol. 2, 53-57